(19) Europäisches Patentamt
European Patent Office
Office européen des brevets

(11) Publication number: **0 261 755 B1**

(12) **EUROPEAN PATENT SPECIFICATION**

(45) Date of publication of patent specification: **16.12.92**

(51) Int. Cl.5: **C07D 307/68**, C07D 333/38, C07D 207/34, C07D 249/04, C07D 249/06, C07D 261/18, C07D 263/16, C07D 275/02, A01N 43/08, A01N 43/10, A01N 43/36

(21) Application number: **87303091.0**

(22) Date of filing: **09.04.87**

(54) **Five-membered heterocyclic derivatives of N'-substituted-N,N'-Diacylhydrazines.**

(30) Priority: **26.09.86 US 911928**

(43) Date of publication of application:
**30.03.88 Bulletin 88/13**

(45) Publication of the grant of the patent:
**16.12.92 Bulletin 92/51**

(84) Designated Contracting States:
**AT BE CH DE ES FR GB GR IT LI LU NL SE**

(56) References cited:
**EP-A- 232 075**
**EP-A- 234 944**
**EP-A- 245 950**
**GB-A- 2 093 448**

(73) Proprietor: **ROHM AND HAAS COMPANY**
**Independence Mall West**
**Philadelphia Pennsylvania 19105(US)**

(72) Inventor: **Chi-Tung Hsu, Adam**
**1686 Heebner Way**
**Lansdale, Pennsylvania 19446,(US)**
Inventor: **Phat Le, Dat**
**125 Washington Avenue**
**North Wales, Pennsylvania, 19454(US)**

(74) Representative: **Angell, David Whilton**
**ROHM AND HAAS (UK) LTD. European Operations Patent Department Lennig House 2**
**Mason's Avenue**
**Croydon CR9 3NB(GB)**

**EP 0 261 755 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with five-membered heterocyclic derivatives of N'-substituted-N,N'-diacylhydrazines which are useful as insecticides, compositions containing those compounds and methods of their use. The disclosed hydrazines are new compounds.

The search for compounds which have a combination of excellent insecticidal activity and low undesirable toxicity is a continuing one because of factors such as the desire for compounds exhibiting greater activity, better selectivity, low undesirable environmental impact, low production cost and effectiveness against insects resistant to many known insecticides.

Compounds of the present invention are particularly suitable for controlling plant-destructive insects in crops of cultivated plants, ornamentals and forestry.

Certain hydrazine derivatives have been disclosed in the literature.

In 25 Aust. J. Chem., 523-529 (1972), several N,N'-dibenzoylhydrazine derivatives are disclosed including N'-i-propyl-; N'-n-propyl-; N'-(2-methylpropyl)-; N'-(3-methylbutyl)-; N'-benzyl- and N'-phenyl-N,N'-dibenzoylhydrazine in which one or both nitrogen atoms are alkylated or phenylated.

In 61 Helv. Chim. Acta, 1477-1510 (1978), several N,N'-dibenzoylhydrazine and hydraside derivatives including N'-t-butyl-N-benzoyl-N'-(4-nitrobenzoyl)-hydrazine are disclosed.

In 44 J.A.C.S., 2556-2567 (1922), isopropylhydrazine $(CH_3)_2CH\text{-}NH\text{-}NH_2$, symmetrical diisopropylhydrazine, dibenzoylisopropylhydrazine and certain derivatives are disclosed.

In 44 J.A.C.S., 1557-1564 (1972), isopropyl, menthyl and bornyl semicarbazides are disclosed.

In 48 J.A.C.S., 1030-1035 (1926), symmetrical di-methylphenylmethylhydrazine and certain related compounds including 1,2-bis-methylphenylmethyl-4-phenyl-semicarbazide are disclosed.

In 27 Bull. Chem. Soc. Japan, 624-627 (1954), certain hydrazine derivatives including alpha,beta-dibenzoylphenylhydrazine are disclosed.

In J. Chem. Soc. (C), 1531-1536 (1966), N,N'-dibenzoylphenylhydrazine and N-acetyl-N'-benzoyl-p-nitrophenylhydrazine are disclosed.

In 56B Chem. Berichte, 954-962 (1923), symmetrical di-isopropylhydrazines, symmetrical diisobutyl- and certain derivatives including N,N'-diisobutyldibenzoylhydrazine are disclosed.

In 590 Annalen der Chemie, 1-36 (1954), certain N,N'-dibenzoylhydrazine derivatives are disclosed including N'-methyl- and N'-(2-phenyl)-isopropyl-N,N'-dibenzoylhydrazine.

In J. Chem. Soc., 4191-4198 (1952), N,N'-di-n-propylhydrazine, N,N'-dibenzoylhydrazine and bis-3,5-dinitrobenzoyl are disclosed.

In 32 Zhur. Obs. Khim., 2806-2809 (1962), N'-2,4-methyl-2,4-pentadiene-N,N'-dibenzoylhydrazine is disclosed.

In 17 Acta. Chim. Scand., 94-102 (1963), 2-benzoylthiobenzhydrazide ($C_6H_5\text{-}CS\text{-}NHNH\text{-}CO\text{-}C_6H_5$) and certain hydrazone and hydrazine derivatives are disclosed including 1,2-dibenzoyl-benzylhydrazine.

In 25 Zhur. Obs. Khim., 1719-1723 (1955), N,N'-bis-cyclohexylhydrazine and N,N'-dibenzoylcyclohexylhydrazine are disclosed.

In J. Chem. Soc., 4793-4800 (1964), certain dibenzoylhydrazine derivatives are disclosed including tribenzoylhydrazine and N,N'-dibenzoylcyclohexylhydrazine.

In 36 J. Prakt. Chem., 197-201 (1967), certain dibenzoylhydrazine derivatives including N'-ethyl-; N'-n-propyl-; N'-isobutyl-; N'-neopentyl-; N'-n-heptyl-; and N'-cyclohexylmethyl-N,N'-dibenzoylhydrazines are disclosed.

In 26 J.O.C., 4336-4340 (1961) N'-t-butyl-N,N'-di-(t-butoxycarbonyl)hydrazide is disclosed.

In 41 J.O.C., 3763-3765 (1976), N'-t-butyl-N-(phenylmethoxycarbonyl)-N'-(chlorocarbonyl)hydrazide is disclosed.

In 94 J.A.C.S., 7406-7416 (1972) N'-t-butyl-N,N'-dimethoxycarbonylhydrazide is disclosed.

In 43 J.O.C., 808-815 (1978), N'-t-butyl-N-ethoyycarbonyl-N'-phenylaminocarbonylhydrazide and N'-t-butyl-N-ethoxycarbonyl-N'-methylaminocarbonylhydrazide are disclosed.

None of the above disclosures teaches any biological activity in relation to these disclosed compounds.

In 39 J. Econ. Ent., 416-417 (1946), certain N-phenyl-N'-acylhydrazines are disclosed and evaluated for their toxicity against codling moth larvae.

The N'-substituted-N,N'-diacylhydrazines of the present invention differ from known compounds primarily by their N'-substituent and their N,N'-diacyl substituents.

Compounds of the present invention are also distinguished by their excellent insecticidal activity against insects of the order Lepidoptera without material adverse impact on beneficial insects.

The invention provides insecticidal compounds which are substituted hydrazines of the formula:

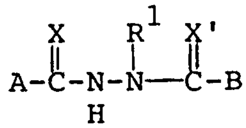

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; alkoxyalkyl having independently 1 to 6 carbon atoms in each alkyl group; alkoxyalkoxy having independently 1 to 6 carbon atoms in each alkyl group; $-OCO_2R$ group; ($C_2$-$C_6$)alkenyl optionally substituted with halo, cyano, ($C_1$-$C_4$)-alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)-alkyl, ($C_1$-$C_4$)alkoxy, halo-($C_1$-$C_4$)alkoxy or ($C_1$-$C_4$)alkylthio; carboxy; $ZCO_2R'$ group; -COR group; halo-($C_1$-$C_6$)alkyl-carbonyl; cyano-($C_1$-$C_6$)alkyl-carbonyl; nitro-($C_1$-$C_6$)alkyl-carbonyl; ($C_1$-$C_6$)alkoxy-carbonyl; halo-($C_1$-$C_6$)alkoxy-carbonyl; -OCOR group; -NRR' group; amino substituted with hydroxy; ($C_1$-$C_4$)alkoxy or ($C_1$-$C_4$)alkylthio groups; -CONRR' group; -OCONRR' group; -NRCOR' group; $-NRCO_2R'$ group; -OCONRCOR' group; sulfhydryl; ($C_1$-$C_6$)alkylthio; halo-($C_1$-$C_6$)-alkylthio; -NRCSR' group; -SCOR group; unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino; benzoyl where the phenyl ring is unsubstituted or substituted with one to three of the same or different halo, cyano, nitro, hydroxy, ($C_1$-$C_4$)-alkyl, ($C_1$-$C_4$)alkoxy, carboxy, ($C_1$-$C_4$)alkoxy-carbonyl, ($C_1$-$C_4$)alkanoyloxy or amino; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy or amino; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino; or when two adjacent positions on the phenyl ring are substituted with alkoxy groups, these groups may be joined to form together with the carbon atoms to which they are attached, a 5 or 6 membered dioxolano or dioxano heterocyclic ring; or

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; $-RCO_2R'$ group; -CONRR' group; -NRR' group; amido -NRCOR' group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, ($C_1$-$C_6$)haloalkoxy, carboxy, ($C_1$-$C_4$)alkoxy-carbonyl or -NRR' group;

where R and R' are hydrogen or ($C_1$-$C_6$)alkyl; Z is ($C_1$-$C_6$)alkyl; "amino" means NRR'; where one of A or B is an unsubstituted or substituted five-membered heterocycle as defined above;

or argonomically acceptable salts thereof.

The invention also provides insecticidal compositions comprising an insecticidal compound of the invention together with agronomically acceptable diluent or carrier and a method of combatting insects which comprises contacting them with such an insecticidal compound or composition.

The term "halo" should be understood as including chloro, fluoro, bromo and iodo. The term "alkyl" by itself or as part of another substituent, unless otherwise stated, includes straight or branched chain groups such as methyl, ethyl, n-propyl, isopropyl, n-butyl, t-butyl, isobutyl, neopentyl and the like and where indicated higher homologues and isomers such as n-octyl, isooctyl and the like. The term "haloalkyl" by itself or as part of another substituent is an alkyl group of the stated number of carbon atoms having one or

more halo atoms bonded thereto such as chloromethyl, 1- or 2-bromoethyl, trifluoromethyl and the like. Analogously, "cyanoalkyl" by itself or as part of another group is an alkyl group of the stated number of carbon atoms having one or more cyano groups bonded thereto; "haloalkoxy" by itself or as part of another group is an alkoxy group of the stated number of carbon atoms having one or more halo atoms bonded thereto such as difluoromethoxy, trifluoromethoxy, 2-fluoroethoxy, 2,2,2-trifluoroethoxy and the like. "Alkenyl" and "alkynyl" by themselves or as part of another substituent comprise straight and branched chain groups of the stated number of carbon atoms. "Alkadienyl" is a straight or branched chain alkenyl group comprising two carbon-carbon double bonds that can be conjugated such as 1,3-butadienyl, cumulated such as 1,2-propadienyl or isolated such as 1,4-pentadienyl. Representative examples of five-membered heterocycles include 2-furyl; 3-furyl; 2-thienyl; 3-thienyl; 4-(1,2,3-triazolyl); 3-(1,2,4-triazolyl); 5-(1,2,4-triazolyl) 2-pyrrolyl; 2-oxazolyl; and the like.

Typical compounds within the scope of the present invention include, but are not limited to:

N'-t-butyl-N-(2-furoyl)-N'-benzoylhydrazine
N'-t-butyl-N-(2-furoyl)-N'-(4-chlorobenzoyl)hydrazine
N'-t-butyl-N-(2-furoyl)-N'-(2,4-dichlorobenzoyl)hydrazine
N'-t-butyl-N-(2-furoyl)-N'-(3-methylbenzoyl)hydrazine
N'-t-butyl-N-(3-furoyl)-N'-(benzoyl)hydrazine
N'-t-butyl-N-(3-furoyl)-N'-(3-methylbenzoyl)hydrazine
N'-t-butyl-N-(3-furoyl)-N'-(3,4-dichlorobenzoyl)hydrazine
N'-t-butyl-N-(2-methyl-3-furoyl)-N'-(benzoyl)hydrazine
N'-t-butyl-N-(3-furoyl)-N'-(2,4-dichlorbenzoyl)hydrazine
N'-t-butyl-N-(3-furoyl)-N'-(4-chlorobenzoyl)hydrazine
N'-t-butyl-N-(3-furoyl)-N'-(2-methylbenzoyl)hydrazine
N'-t-butyl-N-(3-chloro-2-furoyl)-N'-(3-methylbenzoyl)hydrazine
N'-i-propyl-N-(3-furoyl)-N'-(4-chlorobenzoyl)hydrazine
N'-t-butyl-N-benzoyl-N'-(2-furoyl)hydrazine
N'-t-butyl-N-(4-methylbenzoyl)-N'-(2-furoyl)hydrazine
N'-t-butyl-N-(2,3-dimethylbenzoyl)-N'-(2-furoyl)hydrazine
N'-t-butyl-N-(4-ethylbenzoyl)-N'-(3-furoyl)hydrazine
N'-t-butyl-N-(4-chlorobenzoyl)-N'-(3-furoyl)hydrazine
N'-t-butyl-N-(benzoyl)-N'-(2-chloro-3-furoyl)hydrazine
N'-i-propyl-N-(4-chlorobenzoyl)-N'-(2-methyl-3-furoyl)hydrazine
N'-t-butyl-N-(2-thiophenecarbonyl)-N'-benzoylhydrazine
N'-t-butyl-N-(2-thiophenecarbonyl)-N'-(4-methylbenzoyl)hydrazine
N'-t-butyl-N-(2-thiophenecarbonyl)-N'-(3-methylbenzoyl)hydrazine
N'-t-butyl-N-(2-thiophenecarbonyl)-N'-(4-chlorobenzoyl)hydrazine
N'-t-butyl-N-(3-bromo-2-thiophencarbonyl)-N'-(3-methylbenzoyl)hydrazine
N'-i-propyl-N-(2-thiophenecarbonyl)-N'-benzoylhydrazine
N'-t-butyl-N-benzoyl-N'-(2-thiophenecarbonyl)hydrazine
N'-t-butyl-N-(4-methylbenzoyl)-N'-(2-thiophenecarbonyl)hydrazine
N'-t-butyl-N-(4-methylbenzoyl)-N'-(2,5-dichloro-3-thiophenecarbonyl)hydrazine
N'-t-butyl-N-(3,4-dichlorobenzoyl)-N'-(2-thiophenecarbonyl)hydrazine
N'-t-butyl-N-(2,6-difluorobenzoyl)-N'-(2-thiophenecarbonyl)hydrazine
N'-t-butyl-N-(3-thiophenecarbonyl)-N'-benzoylhydrazine
N'-t-butyl-N-(3-thiophenecarbonyl)-N'-(3-methylbenzoyl)hydrazine
N'-t-butyl-N-(3-thiophenecarbonyl)-N'-(3,4-dichlorobenzoyl)hydrazine
N'-t-butyl-N-(3-thiophenecarbonyl)-N'-(4-chlorobenzoyl)hydrazine
N'-t-butyl-N-(N-methyl-2-pyrrolecarbonyl)-N'-benzoylhydrazine
N'-t-butyl-N-(N-methyl-2-pyrrolecarbonyl)-N'-(4-chlorobenzoyl)hydrazine
N'-t-butyl-N-(N-methyl-2-pyrrolecarbonyl)-N'-(3-methylbenzoyl)hydrazine
N'-t-butyl-N-benzoyl-N'-(N-methyl-2-pyrrolecarbonyl)hydrazine
N'-t-butyl-N-benzoyl-N'-(1,2,3-triazole-4-carbonyl)hydrazine
N'-t-butyl-N-(4-methylbenzoyl)-N'-(1,2,3-triazole-4-carbonyl)hydrazine

Insecticidal compounds of the present invention having very good activity for use in the insecticidal compositions and formulations of the present invention include those where any one or combination of two or more of the substituents conforms to the following definitions:

X and X' are O or S;
$R^1$ is branched ($C_3$-$C_8$)alkyl; and/or

A and B are unsubstituted or substituted phenyl having one to three of the same or different halo; nitro; cyano; $(C_1-C_4)$alkyl; halo-$(C_1-C_4)$alkyl; cyano-$(C_1-C_4)$alkyl; $(C_1-C_4)$alkoxy; alkoxyalkyl having independently 1 to 4 carbon atoms in each alkyl group; -COD; $(C_1-C_4)$alkoxy-carbonyl; $(C_1-C_4)$alkanoyloxy; unsubstituted or substituted phenyl having one or two of the same or different halo, nitro $(C_1-C_4)$alkyl, $(C_1-C_4)$alkoxy, carboxy, $(C_1-C_4)$alkoxy-carbonyl, $(C_1-C_4)$alkanoyloxy or -NDD' or phenoxy where the phenyl ring is unsubstituted or substituted with one or two of the same or different halo, nitro, $(C_1-C_4)$alkyl, $(C_1-C_4)$alkoxy, carboxy, $(C_1-C_4)$alkoxy-carbonyl, $(C_1-C_4)$alkanoyloxy or -NDD'; or
unsubstituted or substituted five-membered heterocycle selected from furyl, thienyl, triazolyl, pyrrolyl, and oxazolyl where the substituents can be one or two of the same or different halo; nitro; $(C_1-C_4)$alkyl; $(C_1-C_4)$alkoxy; -NDD'; or unsubstituted or substituted phenyl having one or two of the same or different halo, nitro, $(C_1-C_4)$alkyl, halo-$(C_1-C_4)$alkyl, $(C_1-C_4)$alkoxy, halo-$(C_1-C_4)$alkoxy, carboxy or -NDD';

where D and D' are hydrogen or $(C_1-C_4)$alkyl; where one of A or B is an unsubstituted or substituted five-membered heterocycle as defined.

Because of their insecticidal activity, preferred compounds of the present invention for use in the insecticidal compositions and formulations of the present invention include those where any one or combination of two or more of the substituents conforms to the following definitions:

X and X' are O;
$R^1$ is branched $(C_4-C_7)$alkyl; and
A and B are phenyl or substituted phenyl where the substituents can be from one to three of the same or different halo, nitro, $(C_1-C_4)$alkyl, $(C_1-C_4)$alkoxy or halo-$(C_1-C_4)$alkyl; or
unsubstituted or substituted five-membered heterocycle selected from furyl, thienyl, pyrrolyl and oxazolyl where the substituents can be one or two of the same or different halo, nitro, $(C_1-C_4)$alkyl or $(C_1-C_4)$alkoxy;

where one of A or B is an unsubstituted or substituted five-membered heterocycle as defined above.

Because of their insecticidal activity, particularly preferred compounds of the present invention for use in the insecticidal compositions and formulations of the present invention include those where any one or combination of two or more of the substituents conforms to the following definitions:

X and X' are O;
$R^1$ is t-butyl, neopentyl (2,2-dimethylpropyl) or 1,2,2-trimethylpropyl; and
A and B are phenyl or substituted phenyl where the substituents can be one or two of the same or different chloro, fluoro, bromo, iodo, nitro, methyl, ethyl, methoxy or trifluoromethyl; or
unsubstituted furyl or thienyl or an unsubstituted or substituted pyrrolyl where the substituent can be $(C_1-C_4)$alkyl

where one of A or B is an unsubstituted or substituted five-membered heterocycle as defined above.

Those N'-substituted-N,N'-diacylhydrazines of Formula I which possess acidic or basic functional groups may be further reacted to form novel salts with appropriate bases or acids. These salts also exhibit pesticidal activity. Typical salts are the agronomically acceptable metal salts, ammonium salts and acid addition salts. Among the metal salts are those in which the metal cation is an alkali metal cation such as sodium, potassium, lithium or the like; alkaline earth metal cation such as calcium, magnesium, barium, strontium or the like; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$ and $R^8$ are independently hydrogen, hydroxy, $(C_1-C_4)$-alkoxy, $(C_1-C_{20})$alkyl, $(C_3-C_8)$alkenyl, $(C_3-C_8)$alkynyl, $(C_2-C_8)$hydroxyalkyl, $(C_2-C_8)$alkoxyalkyl, $(C_2-C_6)$-aminoalkyl, $(C_2-C_6)$haloalkyl, amino, $(C_1-C_4)$alkyl- or di-$(C_1-C_4)$alkylamino, substituted or unsubstituted phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, $(C_1-C_8)$alkyl, $(C_1-C_4)$alkoxy, hydroxy, nitro, trifluoromethyl, cyano, amino, $(C_1-C_4)$alkylthio and the like. Such substituted phenyl groups preferably have up to two such substituents. Representative ammonium cations include ammonium, dimethylammonium, 2-ethylhexylammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxy-ethyldimethyloctadecylammonium, 2-hydroxyethyldiethyloctylammonium, decyltrimethylammonium, hexyltriethylammonium, 4-methylbenzyltrimethylammonium, and the like. Among the acid addition salts are those in which the anion is an agronomically acceptable anion such as hydrochloride, hydrobromide, sulfate, nitrate, perchlorate, acetate, oxalate and the like.

The compounds of this invention or their precursors can be prepared according to the following processes.

Process A

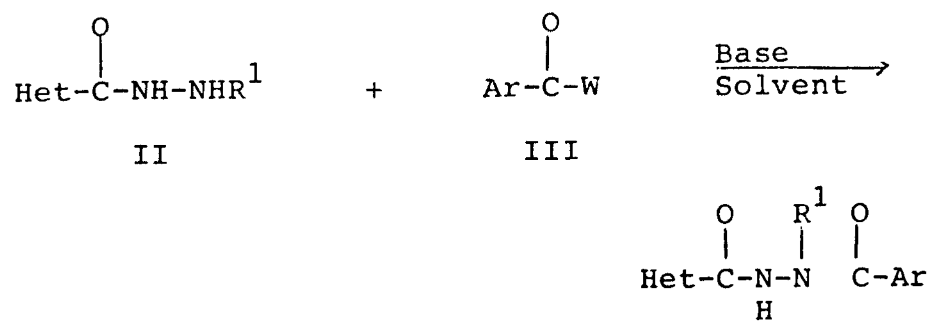


II + III → I

where Het is a five-membered heterocycle as defined above for Formula I, Ar is phenyl as defined above for Formula I, $R^1$ is as defined above for Formula I and W is a good leaving group such as halo, for example, chloro; an alkoxy, for example, ethoxy; a methyl sulfonate ($-0S0_2-CH_3$); or an ester, for example, acetate ($-OC(O)CH_3$).

In Process A, a compound of Formula II is reacted with a compound of Formula III in the presence of a base in an inert or substantially inert solvent or mixture of solvents to afford the desired product of Formula I.

Examples of the compounds of Formula III which can be used in the above process A include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride, methyl benzoate, ethyl benzoate, benzoic acetic anhydride, benzoic methanesulfonic anhydride, and the like. The compounds of Formula III are generally commercially available or can be prepared by known procedures.

Suitable solvents for use in the above process A include water; hydrocarbons such as toluene, xylene, hexane, heptane and the like; alcohols such as methanol, ethanol, isopropanol and the like; glyme, tetrahydrofuran; acetonitrile; pyridine; or haloalkanes such as methylene chloride; or mixtures of these solvents.

Preferred solvents are water, toluene, methylene chloride or a mixture of these solvents.

Examples of bases for use in the above Process A include tertiary amines such as triethylamine; pyridine; potassium carbonate; sodium carbonate; sodium bicarbonate; sodium hydroxide; or potassium hydroxide. Preferred bases are sodium hydroxide, or triethylamine.

The compounds of Formula II are prepared from commercially available compounds by procedures well known to those skilled in the art as described below.

The above Process A can be carried out at temperatures between about -50°C and about 150°C. Preferably, when W is a halo radical, the reaction is carried out between about 0°C and about 30°C. When W is alkoxy, the reaction is preferably carried out between about 100°C and about 150°C. When W is methyl sulfonate, the reaction is preferably carried out between about -20°C to about 20°C. When W is an ester, the reaction is preferably carried out between about 0°C and about 50°C.

Preparation of the compounds of the present invention by Process A is preferably carried out at about atmospheric pressure, although higher or lower pressures can be used if desired.

Substantially equimolar amounts of reactants are preferably used in Process A, although higher or lower amounts can be used if desired.

Generally, about one equivalent of base is used per equivalent of the reactant of Formula III.

Process B

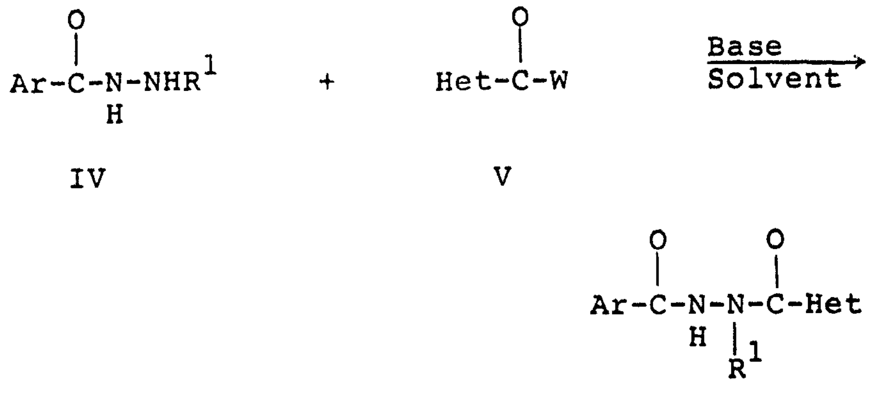


IV

V

I

where Ar is phenyl as defined above for Formula I, $R^1$ is as defined above for Formula I, Het is a five-membered heterocycle as defined above for Formula I and W is a good leaving group such as halo, for example, chloro; an alkoxy, for example, ethoxy; methyl sulfonate ($-OSO_2CH_3$); or an ester, for example, acetate ($-OC(O)CH_3$).

In Process B, an N'-substituted-N-benzoylhydrazine of Formula IV is reacted with a compound of Formula V in the presence of a base in an inert or substantially inert solvent or mixture of solvents to afford the desired product of Formula I.

Examples of the compounds of Formula IV which can be used in the above process B include N'-isopropyl-N-benzoylhydrazine; N'-sec-butyl-N-benzoylhydrazine; N'-(1-methyl)neopentyl-N-benzoylhydrazine; N'-neopentyl-N-benzoylhydrazine; N'-isobutyl-N-benzoylhydrazine; N'-(1,2,2-trimethylpropyl)-N-benzoylhydrazine; N'-diisopropylmethyl-N-benzoylhydrazine; N'-t-butyl-N-benzoylhydrazine; N'-t-butyl-N-(4-methylbenzoyl)hydrazine; N'-t-butyl-N-(4-chlorobenzoyl)hydrazine; and the like.

The compounds of Formula V are generally commercially available or are prepared from commercially available compounds by procedures well known to those skilled in the art as described below.

Suitable solvents for use in the above Process B include water; hydrocarbons such as toluene, xylene, hexane, heptane and the like; alcohols such as methanol, ethanol, isopropanol and the like; glyme, tetrahydrofuran; acetonitrile; pyridine; or haloalkanes such as methylene chloride; or mixtures of these solvents. Preferred solvents are water, toluene, methylene chloride or a mixture of these solvents.

Examples of bases suitable for use in the above Process B include tertiary amines such as triethylamine; pyridine; potassium carbonate; sodium hydroxide; or potassium hydroxide. Preferred bases are sodium hydroxide, or triethylamine.

The above Process B can be carried out at temperatures between about -50 °C and about 150 °C. Preferably, when W is a halo radical, the reaction is carried out between about 0 °C and about 30 °C. When W is alkoxy, the reaction is preferably carried out between about 100 °C and about 150 °C. When W is methyl sulfonate, the reaction is preferably carried out between about -20 °C to about 20 °C. When W is an ester, the reaction is preferably carried out between about 0 °C and about 50 °C.

Preparation of the compounds of the present invention by Process B is preferably carried out at about atmospheric pressure, although higher or lower pressures can be used if desired.

Substantially equimolar amounts of reactants are preferably used in Process B, although higher or lower amounts can be used, if desired.

Generally, about one equivalent of base is used per equivalent of the reactant of Formula V.

Process C

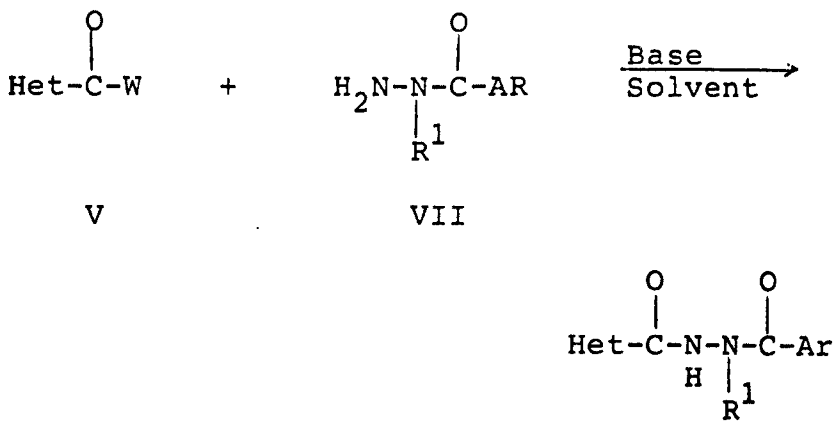


V VII

I

where Ar is phenyl as defined above for Formula I, Het is a five-membered heterocycle as defined above for Formula I, $R^1$ is as defined above for Formula I and W is a good leaving group such as halo, for example, chloro; an alkoxy, for example, ethoxy; methyl sulfonate ($-OSO_2CH_3$); or an ester, for example, acetate ($-OC(O)CH_3$).

In Process C, an N'-substituted-N'-benzoylhydrazine of Formula VII is reacted with a compound of Formula V in the presence of a base in an inert or substantially inert solvent or mixture of solvents to afford the desired product of Formula I.

The compounds of Formula V are generally commercially available or can be prepared from commercially available compounds by procedures well known to those skilled in the art as described below.

Examples of the compounds of Formula VII which can be used in the above Process C include N'-t-butyl-N'-benzoylhydrazine; N'-t-butyl-N'-(3-methylbenzoyl)hydrazine; N'-t-butyl-N'-(4-chlorobenzoyl)-hydrazine; N'-t-butyl-N'-(2-fluorobenzoyl)hydrazine; N'-isopropyl-N'-benzoylhydrazine; N'-neopentyl-N'-(4-chlorobenzoyl)hydrazine, and the like.

Suitable solvents for use in the above Process C include water; hydrocarbons such as toluene, xylene, hexane, heptane and the like; alcohols such as methanol, ethanol, isopropanol and the like; glyme; tetrahydrofuran; acetonitrile; pyridine; or haloalkanes such as methylene chloride; or mixtures of these solvents. Preferred solvents are water, toluene, methylene chloride or a mixture of these solvents.

Examples of bases suitable for use in the above Process C includes tertiary amines such as triethylamine; pyridine; potassium carbonate; sodium carbonate; sodium bicarbonate; sodium hydroxide; or potassium hydroxide. Preferred bases are sodium hydroxide, or triethylamine.

The above Process C an be carried out at temperatures between about -50°C and about 150°C. Preferably, when W is a halo radical, the reaction is carried out between about 0°C and about 30°C. When W is alkoxy, the reaction is preferably carried out between about 100°C and about 150°C. When W is methyl sulfonate, the reaction is preferably carried out between about -20°C to about 20°C. When W is an ester, the reaction is preferably carried out between about 0°C and about 50°C.

Preparation of the compounds of the present invention by Process C is preferably carried out at about atmospheric pressure, although higher or lower pressures can be used if desired.

Substantially equimolar amounts of reactants are preferably used in Process C, although higher or lower amounts can be used if desired.

Generally, about one equivalent of base is used per equivalent of the reactant of Formula V.

The compounds of Formula II are prepared by procedures well known to those skilled in the art. By way of examples, a suitably substituted hydrazine (such as t-butylhydrazine) is reacted with a heterocyclic ester (such as) in an inert or substantially inert solvent or mixture of solvents (such as ethanol), with heat, to afford the compounds of Formula II (such as N'-t-butyl-N-hydrazine; a heterocycle carboxylic acid (such as 2-) is reacted with methanesulfonyl chloride in the presence of a base (such as triethylamine) in an inert or substantially inert solvent or mixture of solvents (such as methylene chloride) to afford the corresponding mixed anhydride (such as) which is then reacted with a suitably substituted hydrazine (such as t-butylhydrazine) in the presence of a base (such as triethylamine) in an inert or substantially inert solvent or mixture of solvents (such as methylene chloride) to afford the compounds of Formula II (such as N'-t-butyl-N-hydrazine); a suitably substituted hydrazine (such as t-butylhydrazine) is reacted with a heterocyclic carboxylic acid halide (such as) in the presence of a base (such as sodium hydroxide) in an inert or substantially inert solvent or mixture of solvents (such as toluene) to afford the compounds of Formula II (such as N'-t-butyl-N-hydrazine).

Suitably substituted hydrazines such as t-butylhydrazine, isopropylhydrazine and the like are commercially available or can be prepared by procedures well known to those skilled in the art.

The compounds of Formula IV are prepared from commercially available materials by procedures known to those skilled in the art. By way of example, a suitably substituted hydrazine (such as t-butylhydrazine) is reacted with a benzoyl chloride (such as benzoyl chloride, 3-methylbenzoyl chloride or 4-chlorobenzoyl chloride) in the presence of a base (such as aqueous sodium hydroxide) in an inert or substantially inert solvent or mixture of solvents (such as toluene) to afford the compounds of Formula IV (such as N'-t-butyl-N-benzoylhydrazide, N'-t-butyl-N-(3-methylbenzoyl)hydrazine or N'-t-butyl-(4-chlorobenzoyl)hydrazine).

The compounds of Formula V are commercially available, such as or can be prepared from commercially available materials by procedures known to those skilled in the art as described above.

The compounds of Formula VII can be prepared by procedures known to those skilled in the art from commercially available reactants. By way of example, a suitably substituted hydrazine (such as t-butylhydrazine) is reacted with an aldehyde or ketone (such as acetone) in the presence of a base (such as triethylamine) to afford a hydrazone which is then reacted with a benzoyl chloride in an inert or substantially inert solvent or mixture of solvents in the presence of a base (such as sodium hydroxide) to afford an N'-substituted-N'-benzoylhydrazone which is then reacted with an acid (such as hydrochloric acid) to afford the compound of Formula VII. Alternatively, a suitably substituted hydrazine (such as t-butylhydrazine) is reacted with di-tert-butyldicarbonate in an inert or substantially inert solvent or mixture of solvents (such as toluene/water) to afford an N'-t-butyl-N-t-butoxycarbonylhydrazine which is then reacted with a benzoylchloride in an inert or substantially inert solvent or mixture of solvents to afford an N'-t-butyl-N'-benzoyl-N-t-butoxycarbonylhydrazine which is then reacted with an acid to afford the desired compound of Formula VII.

Modifications to the above processes may be necessary to accommodate reactive functionalities of particular A and/or B substituents. Such modifications would be apparent and known to those skilled in the art.

It will be appreciated by those skilled in the art that electronic attractive forces may give rise to more than one isomer of the compounds of Formula I. There may be a difference in properties such as biological activity and physical characteristics between such isomers. It is believed the procedures for making the compounds of Formula I described herein will not preferentially afford one isomer over another. Separation of a specific isomer can be accomplished by standard techniques well known to those skilled in the art such as silica gel chromatography.

The agronomically acceptable salts of the compounds embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt of a compound of Formula I in a suitable solvent. When metal hydroxides are used as reagents, useful solvents include water; ethers such as glyme and the like; dioxane; tetrahydrofuran; alcohols such as methanol, ethanol, isopropanol and the like. When metal hydrides are used as reagents, useful solvents include nonhydroxylic solvents, for example, ethers such as dioxane, glyme, diethylether and the like; tetrahydrofuran; hydrocarbons such as toluene, xylene, hexane, pentane, heptane, octane and the like; dimethylformamide, and the like. When amines are used as reagents, useful solvents include alcohols, such as methanol or ethanol; hydrocarbons, such as toluene, xylene, hexane and the like; tetrahydrofuran; glyme; dioxane; or water. When ammonium salts are used as reagents, useful solvents include water; alcohols, such as methanol or ethanol; glyme; tetrahydrofuran; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0 °C to about 100 °C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones, haloalkanes and the like. The particular choice of solvent will depend on the relative solubilities of the starting materials and the resulting salts and slurries rather than solutions of certain reagents may be

used to obtain the salts. Generally, equivalent molar amounts of starting materials are used and the salt-forming reaction is carried out at from about -10°C to about 100°C, preferably at about room temperature.

The following examples will further illustrate this invention but are not intended to limit it in any way. In Table I, six-membered heterocyclic derivatives of some N'-substituted-N,N'-diacyl hydrazines of the present invention that have been made are listed. The structure of these compounds was confirmed by NMR and in some cases by IR and/or elemental analysis. Specific illustrative preparation of the compounds of Examples 1, 2, 3, 4, 9, 15 and 24 are described after Table I.

TABLE I

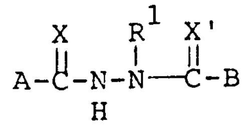

| Ex. No. | X | X' | $R^1$ | A | B |
|---|---|---|---|---|---|
| 1 | O | O | $-C(CH_3)_3$ | $C_6H_5$ | 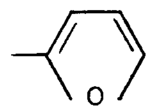 |
| 2 | O | O | $-C(CH_3)_3$ | $C_6H_5$ | 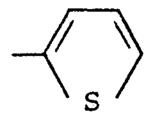 |
| 3 | O | O | $-C(CH_3)_3$ | 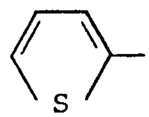 | $C_6H_5$ |
| 4 | O | O | $-C(CH_3)_3$ | 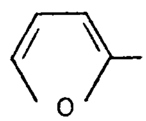 | $C_6H_5$ |
| 5 | O | O | $-C(CH_3)_3$ | $-C_6H_4CH_3-3$ | 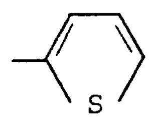 |
| 6 | O | O | $-C(CH_3)_3$ | 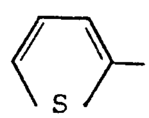 | $-C_6H_4CH_3-4$ |
| 7 | O | O | $-C(CH_3)_3$ | 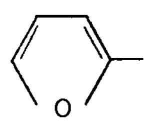 | $-C_6H_6CH_3-3$ |
| 8 | O | O | $-C(CH_3)_3$ | $-C_6H_4CH_3-4$ | 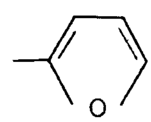 |
| 9 | O | O | $-C(CH_3)_3$ | $-C_6H_4CH_3-4$ | 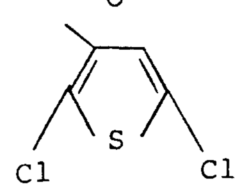 |

| Ex. No. | X | X' | $R^1$ | A | B |
|---|---|---|---|---|---|
| 10 | O | O | $-C(CH_3)_3$ | $-C_6H_3Cl_2$-3,4 | 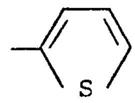 |
| 11 | O | O | $-C(CH_3)_3$ | 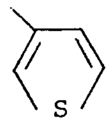 | $C_6H_5$ |
| 12 | O | O | $-C(CH_3)_3$ | 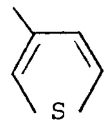 | $-C_6H_4CH_3$-3 |
| 13 | O | O | $-C(CH_3)_3$ | 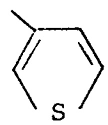 | $-C_6H_3Cl_2$-3,4 |
| 14 | O | O | $-C(CH_3)_3$ | 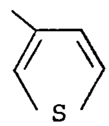 | $-C_6H_4Cl$-4 |
| 15 | O | O | $-C(CH_3)_3$ | 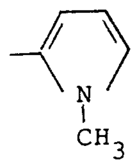 | $C_6H_5$ |
| 16 | O | O | $-C(CH_3)_3$ | 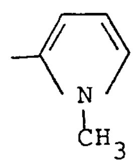 | $-C_6H_4Cl$-4 |
| 17 | O | O | $-C(CH_3)_3$ | 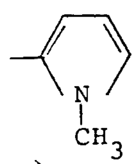 | $-C_6H_4CH_3$-3 |
| 18 | O | O | $-C(CH_3)_3$ | 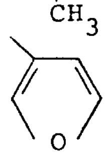 | $C_6H_5$ |
| 19 | O | O | $-C(CH_3)_3$ | 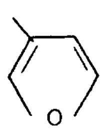 | $-C_6H_4CH_3$-2 |
| 20 | O | O | $-C(CH_3)_3$ | 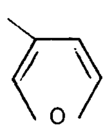 | $-C_6H_4Cl$-4 |

| Ex. No. | X | X' | $R^1$ | A | B |
|---|---|---|---|---|---|
| 21 | O | O | $-C(CH_3)_3$ | 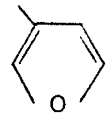 | $-C_6H_3Cl_2-2,4$ |
| 22 | O | O | $-C(CH_3)_3$ | 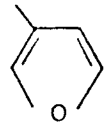 | $-C_6H_4CH_3-3$ |
| 23 | O | O | $-C(CH_3)_3$ | $-C_6H_3F_2-2,6$ | 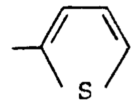 |
| 24 | O | O | $-C(CH_3)_3$ | 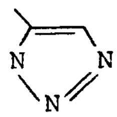 | $-C_6H_4CH_3-3$ |
| 25 | O | O | $-C(CH_3)_3$ | $-C_6H_4Cl-2-OCH_3-3$ | 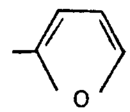 |
| 26 | O | O | $-C(CH_3)_3$ | 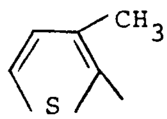 | $-C_6H_4CH_3-3$ |
| 27 | O | O | $-C(CH_3)_3$ | $C_6H_5$ | 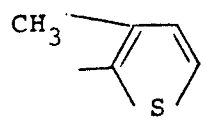 |
| 28 | O | O | $-C(CH_3)_3$ | 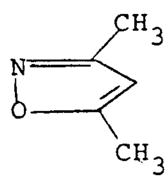 | $C_6H_5$ |
| 29 | O | O | $-C(CH_3)_3$ | $-C_6H_4CH_2CH_3-4$ | 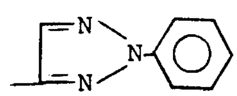 |
| 30 | O | O | $-C(CH_3)_3$ | $C_6H_5$ | 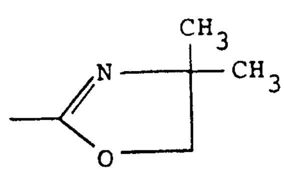 |
| 31 | O | O | $-C(CH_3)_3$ | 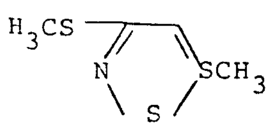 | $C_6H_5$ |

EXAMPLE 1 - Preparation of N'-t-butyl-N-benzoyl-N'-(2-furoyl)hydrazine

N'-t-butyl-N-benzoylhydrazine (1 g) was dissolved in 20 ml toluene. Water (5 ml) and 50% aqueous sodium hydroxide (1.25 g) was added followed by 2-furoyl-chloride (0.68 g). After stirring for 7 hours at room temperature, 0.3 g 2-furoylchloride was added and the mixture stirred for a further 6 hours. The solid product, N'-t-butyl-N-benzoyl-N'-(2-furoyl)hydrazine, was removed by filtration and washed with water. m.p. 155-175 °C.

## EXAMPLE 2 - Preparation of N'-t-butyl-N-benzoyl-N'-(2-thiophenecarbonyl)hydrazine

N'-t-butyl-N-benzoylhydrazine (1.0 g) was dissolved in 20 ml toluene. 50% aqueous sodium hydroxide (1.25 g) was added followed by 2-thiophenecarbonylchloride (0.76 g). The mixture was stirred at room temperature for 14 hours. The solid product, N'-t-butyl-N-benzoyl-N'-(2-thiophenecarbonyl)hydrazine, was removed by filtration and washed with water. m.p. >200 °C.

## EXAMPLE 3 - Preparation of N'-t-butyl-N-(2-thiophenecarbonyl)-N'-benzoylhydrazine

N'-t-butyl-N-(2-thiophenecarbonyl)hydrazine (1 g) was dissolved in 10 ml toluene and treated with 50% aqueous sodium hydroxide (1.0 g) and water (2 ml) followed by benzoylchloride (0.8 g). After stirring for 14 hours at room temperature, the solid product, N'-t-butyl-N-(2-thiophenecarbonyl)-N'-benzoylhydrazine, was removed by filtration and washed with water. m.p. >190 °C.

## EXAMPLE 4 - Preparation of N'-t-butyl-N-(2-furoyl)-N'-benzoylhydrazine

N'-t-butyl-N-(2-furoyl)hydrazine (1.0 g) was dissolved in 10 ml toluene and 2 ml water. 50% aqueous sodium hydroxide (1.0 g) was added followed by benzoylchloride (0.8 g). After stirring for 14 hours at room temperature, the solid product, N'-t-butyl-N-(2-furoyl)-N'-benzoylhydrazine, was removed by filtration and washed with water. m.p. 160-162 °C.

## EXAMPLE 9 - Preparation of N'-t-butyl-N-(4-methylbenzoyl)-N'-(2,5-dichlorothiophene-3-carbonyl)-hydrazine

N'-t-butyl-N-4-methylbenzoylhydrazine (0.7 g) was dissolved in 35 ml toluene. Water (5 ml) and 50% aqueous sodium hydroxide (0.8 g) were added followed by 2,5-dichlorothiophene-3-carbonylchloride (2.0 g). After stirring for 3 hours at room temperature, ether was added and the organic layer separated. Evaporation afforded a solid which was triturated with 10% ether-hexane to afford N'-t-butyl-N-(4-methylbenzoyl)-N'-(2,5-dichlorothiophene-3-carbonyl)hydrazine. m.p. 163-165 °C.

## EXAMPLE 15 - Preparation of N'-t-butyl-N-(N-methyl-2-pyrrolecarbonyl)-N'-benzoylhydrazine

N'-t-butyl-N-(N-methyl-2-carbonylpyrrole)hydrazine (0.8 g) was dissolved in 10 ml toluene and 1 ml water. 50% aqueous sodium hydroxide (10 drops) was added followed by benzoylchloride (1.2 g). After 14 hours stirring at room temperature, ether was added and the product, N'-t-butyl-N-(N-methyl-2-pyrrolecarbonyl)-N'-benzoylhydrazine, was isolated by filtration and washed with ether. m.p. 182-185 °C.

## EXAMPLE 24 - Preparation of N'-t-butyl-N-(1,2,3-triazole-4-carbonyl)-N'-(3-methylbenzoyl)hydrazine

1,2,3-triazole-4-carboxylic acid (1.0 g) and triethylamine (0.9 g) were dissolved in 40 ml methylene chloride and cooled in an ice bath. Methanesulfonylchloride (1.0 g) was added dropwise. After addition was complete, the reaction mixture was stirred for 0.5 hours. N'-t-butyl-N'-(3-methylbenoyl)hydrazine (1.84 g) in 10 ml $CH_2Cl_2$ was added dropwise. The resulting mixture was allowed to stand for 14 hours. Aqueous sodium bicarbonate was added. The organic layer was dried over anhydrous magnesium sulfate, filtered and evaporated to give a yellow oil. Chromatography on silica gel using acetone was eluant afforded N'-t-butyl-N-1,2,3-triazole-4-carbonyl-N'-(3-methylbenzoyl)hydrazine.

By following substantially the procedures in the processes described above and as exemplified by the preparation of the compounds of Examples 1, 2, 3, 4, 9, 15 and 24, the compounds of Formula I are prepared.

As previously noted, the compounds of the present invention exhibit excellent insecticidal activity and are selective against insects of the order Lepidoptera.

In general, for the control of insects in agriculture, horticulture and forestry, the compounds of the present invention may be used at a dosage corresponding to from about 10 grams to about 10 kilograms of the active substance per hectare and from about 100 grams to about 5 kilograms per hectare of the active substance is preferred. The exact amount of dosage for a given situation can be routinely determined and depends on a variety of factors, for example, the substance used, the kind of insect, the formulation used, the state of the crop infested with the insect and the prevailing weather conditions. The term "insecticidal" as employed in the specification and claims of this application is to be construed as any means which adversely affects the existence or growth of the target insects. Such means can comprise a complete killing action, eradication, arresting in growth, inhibition, reducing in number or any combination thereof. The terms "control" and "combat" as employed in the specification and claims of this application is to be construed as including "insecticidal" and protection of plants from insect damage. By "insecticidally effective amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research", (1969), Advances in Chemistry Series No. 86, written by Wade Van Valkenburg; and the Marcel Dekker, Inc. publication "Pesticide Formulations", (1973), edited by Wade Van Valkenburg. In these compositions and formulations, the active substance or substances are mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, conventional adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behaviour, feeding habits and physiology of the pest for the purpose of control.

The invent emulsions are mainly used for air application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistance, e.g., conventional surface-active agents, including emulsifying agents and/or dispersing agents, whereby, for example, in the case where water is used as diluent, organic

solvents may be added as auxiliary solvents. The following may be chiefly considered for use as conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.,), alcohols (e.g., methanol, ethanol, propanol, butanol, glycol, etc.) as well as ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl isobutyl ketone, cyclohexanone, isophorone, etc.), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.); and/or dispersing agents, such as lignin, sulfite waste liquors, methyl cellulose, etc.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists, etc., if desired, or in the form of particular dosage preparations for specific application made therefrom, such as solutions, emulsions, suspensions, powders, pastes, and granules which are thus ready for use.

As concerns commercially marketed preparations, these generally contemplate carrier composition mixtures in which the active compound is present in an amount substantially between about 0.1% and 99% by weight, preferably between about .1% and 90% by weight, and more preferably between about 1% and 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible diluent or carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally, between about 0.0001% and about 99% by weight of the composition, preferably between about 0.001% and about 90% by weight of the composition, and more preferably between about 0.01% and about 75% by weight of the composition, which is effective for the purpose in question.

The active compounds can be applied as sprays by methods commonly employed, such as conventional high-gallonage hydraulic sprays, low-gallonage sprays, ulta-low-volume sprays, airblast spray, aerial sprays, and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions

with said liquid carrier vehicles containing from about 20 to about 95% by weight of the active compound.

Furthermore, the present invention contemplates methods of killing, combatting or controlling insects, which comprises contacting insects with a correspondingly combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or to an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example, as the lignosulfonates or alkylnaphthalene sulfonates known for this purpose and preforably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e. preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

Representative preparation of compositions and formulations including the compounds of the present invention are set forth below as Examples A through I by way of illustration but not limitation.

Example A

Granular

| Ingredient | %/wt. |
|---|---|
| Toxicant and toxicant impurities | 0.25 |
| Triton X-305 (binder) (Octylphenyl-30-ethylene oxide ethanol) | 0.25 |
| Agsorb 24/48 (diluent) (Montmorillonite clay) | 99.50 |

Preparation: The toxicant and Triton X-305 are dissolved into methylene chloride and the mixture is added to the Agsorb with continuous mixing. The methylene chloride is then allowed to evaporate.

Example B

Dust

| Ingredient | %/wt. |
|---|---|
| Toxicant and toxicant impurities | 1.0 |
| Talc | 99.0 |

Preparation: Toxicant is dissolved in excess acetone and the mixture is impregnated onto the talc. The acetone is then permitted to evaporate.

Example C

Wettable Powder

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HiSil 233 (diluent) (Sodium silica) | 30.0 |

Preparation: The toxicant, optionally dissolved in a volatile solvent, is absorbed onto the Barden clay and HiSil carriers. The Duponal and Reax are then added and the entire dry mixture blended until homogeneous. The composition is then micronized to a fine particle size.

Example D

Emulsifiable Concentrate

| Ingredient | %/wt. |
|---|---|
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecy benzene sulfonate; and ethoxylated alkylphenol) | 4.0 |
| Cyclohexanone (solvent) | 22.5 |
| Tenneco 500-100 (solvent) (Aromatic solvent mixture) principally comprising xylene, cumene and ethyl benzene having a boiling point range of 290-345° F) | 52.5 |

Preparation: All ingredients are mixed together with continuous agitation until a homogeneous clear solution is obtained.

EXAMPLE E

Aerosol

| Ingredient | %/wt. |
|---|---|
| Toxicant and toxicant impurities | 0.5 |
| Freon 12 | 99.5 |

Preparation: The components are mixed and packaged under pressure in a suitable container equipped with a release spray valve.

EXAMPLE F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

EXAMPLE G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 (preservative) (2-n-octyl-4-isothiazolin-3-one) | 0.05 |

Preparation: The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example H

Pellet

Same as Example G, Method A, with this addition: the bait composition is formed into 0.635 cm (1/4") diameter by 0.95 cm (3/8") long pellets using a suitable die and press apparatus.

Example I

Flowable

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| HiSil 233 (diluent) (Sodium silica) | 30.0 |
| Kelzan (thickener) (Xanthan gum) | 0.5 |
| Water | 31.2 |

Preparation: The toxicant is absorbed onto the HiSil carrier. The Duponal and Reax are then added and the entire dry mixture blended until homogeneous. The composition is then micronized to a fine particle size. The resulting powder is suspended in water and the Kelzan added.

Compositions and formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis(p-chlorophenyl)-1,1,1-trichloroethane and hexachloroepoxyoctahydrodimethanonaphthalene;

Carbamates, for example, N-methyl-1-naphthylcarbamates;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, 0,0-diethyl-O-p-nitrophenylphosphorothioate; N-monomethylamide of O,O-dimethyldithiophosphorylacetic acid;

Diphenylsulfides, for example, p-chlorobenzyl or p-chlorophenyl sulfide and 2,4,4',5-tetrachlorodiphenylsulfide;

Diphenylsulfonates, for example, p-chlorophenylbenzenesulfonate;

Methylcarbinols, for example, 4,4-dichloro-1-trichloromethylbenzhydrol;

Quinoxaline compounds, such as methylquinoxaline dithiocarbonate;

Amidines such as N'-(4-chloro-2-methylphenyl) N,N-dimethylformamidine;

Pyrethroids such as Allethrin;

Biologicals such as Bacillus thuringiensis preparations;

Organic tin compounds such as tricyclohexyltin hydroxide;
Synergists such as piperonyl butoxide.
Fungicides such as:
Organic mercury compounds, for example, phenylmercuryacetate and methylmercurycyanoguanide;
Organic tin compounds, for example, triphenyltin hydroxide and triphenyltin acetate;
Alkylenebisdithiocarbamates, for example, zinc ethylenebisthiocarbamate and manganese ethylenebisdithiocarbamate; and

2,4-dinitro-6-(2-octyl-phenylcrotonate), 1- bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

## Biological Activity

It has been found by biological evaluation that compounds according to the present invention have pesticidal activity and are capable of controlling larvae and adult forms of pests, especially insects from the order Lepidoptera. One skilled in the art will know how to determine the activity of a given compound against a given insect and the dosage required to obtain general or selective insecticidal effects. The compounds of the present invention in part affect the normal development of insects, particularly insects from the order Lepidoptera, by directly and/or indirectly influencing the moulting process.

As previously noted, the compounds of the present invention are particularly suitable for controlling plant destructive insects in crops of cultivated plants, such as, but not limited to, cotton, vegetables, corn and other cereals and the like;

forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to fruit and/or citrus trees, raspberry bushes and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton X-155) and a modified phthalic glycerol alkyl resin (sold under the trademark Triton B-1956) was utilized at the equivalent of 1 ounce per 100 gal. of test solution as a surfactant.

Initial evaluations were made on one or more of the following pests:

| Code Symbol | Common Name | Latin Name |
|---|---|---|
| SAW | Southern Armyworm | Spodoptera eridania |
| MBB | Mexican Bean Beetle | Epilachna varivestis |
| BW | Boll Weevil | Anthonomus grandis grandis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

For the Boll Weevil test ten adult weevils are placed in a 0.24 l (0.5 pint) glass Mason jar containing a small cube of apple. The weevils are confined to the jars by fiberglass screen mesh secured by a screw-type rim cap. The jars are then sprayed with the test solution using a rotating turntable, directing the spray through the mesh into the jar.

Percent mortalities for the bean beetle and armyworm evaluations are determined 96 hours after treatment. Boll weevil mortality is determined 48 after treatment. Evaluations are based on a scale of 0-100 percent in which 0 equals no activity and 100 equals total kill.

The rotating turntable consists of a fixed, continuously operating spray nozzle under which targets are rotated at a fixed speed and distance. If the target is a Petri dish (such as for the armyworm), the distance from the nozzle is 38.1 cm (15 inches). If the target is a Mason jar, the distance between the screened lid and the nozzle is 15.24 cm (6 inches) (25.4 cm (10 inches) from the base of the jar to the nozzle). The

nozzle is located 20.32 cm (8 inches) from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 1.7 bar (10 psig) air pressure used and with liquid siphon feed 1.9 1/hour (0.5 gallons per hour) are delivered in a round spray pattern with a 21° spray angle. Targets are misted with spray droplets to the point that the droplets coalesce to form a uniform thin film insufficient to drown test organisms.

All treatments are maintained at 21.1-26.7°C (75-80°F) under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 7.62 cm (3-inch) pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control (mortality) is determined. A second observation may be made 6 days after infesting the dishes if the experimenter feels the effect may not be complete or moribund insects appear to evidence signs of some recovery. Where necessary, untreated bean leaves are introduced into dishes held for a second observation to preclude insect starvation.

The results of the initial insecticidal evaluations are given in Table II.

Armyworm and bean beetle spray (foliar) results are 96 hour observations. Boll weevil spray results are 48 hour observations. Soil treatment results are 72 hour observations. At the discretion of the experimenter, particular evaluations were held for 144 hour observations. If, after 144 hours, there was a change in the percent control, it is shown in parenthesis.

TABLE II

Initial Biological Evaluations

| Example No. | Foliar Application Test Species SAW | MBB | BW | Soil Application Test Species MBB | SAW |
|---|---|---|---|---|---|
| 1 | 80 | 80 | 0 | 0 | 50(90) |
| 2 | 100 | 10 | 0 | 40(100) | 100 |
| 3 | 100 | 20 | 20 | 0(20) | 100 |
| 4 | 100 | 0 | 0 | 0 | 0 |
| 5 | 50 | 10 | 40 | 0 | 0 |
| 6 | 100 | 20 | 20 | 0(20) | 0 |
| 7 | 50 | 30 | 20 | 0(20) | 0 |
| 8 | 0 | 0 | 0 | 0(20) | 0 |
| 9 | 90 | 10 | 0 | 0(20) | 0 |
| 10 | 20 | 40 | 0 | 0 | 0 |
| 11 | 100 | 0 | 20 | 20 | 90 |
| 12 | 100 | 30 | 0 | 0 | 20(30) |
| 13 | 40 | 0 | 0 | 0 | 0 |
| 14 | 0 | 0 | 0 | 0 | 0 |
| 15 | 100 | 10 | 0 | 0 | 0 |
| 16 | 90 | 20 | 0 | 0 | 0 |
| 17 | 100 | 0 | 0 | 0 | 0 |
| 18 | 100 | 80 | 0 | 40(100) | 100 |
| 19 | 40 | 70 | 0 | 0(20) | 40 |
| 20 | 100 | 10 | 0 | 0 | 10 |
| 21 | 100 | 30 | 0 | 0 | 0 |
| 22 | 100 | 20 | 0 | 40(0) | 80(100) |
| 23 | 60 | 0 | 0 | 0 | 0 |
| 24 | 30 | 30 | 0 | n.t. | n.t. |
| 25 | n.t. | n.t. | n.t. | n.t. | n.t. |
| 26 | n.t. | n.t. | n.t. | n.t. | n.t. |

TABLE II (cont'd)

Initial Biological Evaluations

| Example No. | Foliar Application Test Species SAW | MBB | BW | Soil Application Test Species MBB | SAW |
|---|---|---|---|---|---|
| 27 | n.t. | n.t. | n.t. | n.t. | n.t. |
| 28 | 0 | 10 | 0 | 0 | 0 |
| 29 | 0 | 0 | 0 | 0 | 0 |
| 30 | 20 | 0 | 0 | n.t. | n.t. |
| 31 | 0 | 10 | 0 | 0 | 0 |

a - 72 hours exposure data. Supplemental 144 hour exposure values, where obtained and different, are given in parentheses.

n.t. = not tested.

Because of their relatively low activities at low application rates, the less preferred compounds of this invention are those in which:

A is 4-alkyl substituted phenyl and B is 2-furyl or
A is 4-(3,5-dimethyl)isoxazolyl or
A is (3,5-dimethylthio-isothiazolyl;

and the least preferred are those in which:

A is 3-thienyl and B is 4-chloro substituted phenyl or
A is 4-alkyl substituted phenyl and B is 4-(2-phenyl)-1,2,4-triazolyl.

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

The following words are trademarks which may be registered in some or all of the designated states: Triton, Agsorb, Duponal, Reax, Hisil, Sponto, Tenneco, Kathon and Kelzan.

**Claims**

**Claims for the following Contracting States : BE, CH, DE, FR, GB, GR, IT, LI, LU, NL, SE**

**1.** An insecticidally active compound which is a substituted diacylhydrazine of the formula

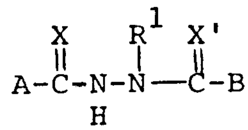

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$c_6$)alkoxy; alkoxyalkyl having independently 1 to 6 carbon atoms in each akyl group; alkoxyalkoxy having independently 1 to 6 carbon atoms in each alkyl group; $-OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)-alkylthio; carboxy; $-ZCO_2R'$ group; -COR'; halo-($C_1$-$C_6$)alkyl-carbonyl; cyano-($C_1$-$C_6$)-alkyl-carbonyl; nitro-($C_1$-$C_6$)alkyl-carbonyl; ($C_1$-$C_6$)alkoxy-carbonyl; halo-($C_1$-$C_6$)alkoxy-carbonyl- -OCOR group; -NRR' group; amino substituted with hydroxy, ($C_1$-$C_4$)alkoxy or ($C_1$-$C_4$)alkylthio groups; -CONRR' group; -OCONRR' group; -NRCOR' group; $-NRCO_2R'$ group; -OCONRCOR' group; sulfhydryl; ($C_1$-$C_6$)alkylthio; halo-($C_1$-$C_6$)alkylthio; -NRCSR' group; -SCOR group;

or unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$) alkanoyloxy or amino;

or phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substitutents can be from one to three of the same or different halo; nitro; hydroxy; ($C_1$-$C_6$)alkyl; ($C_1$-$C_6$)aloxy; carboxy; ($C_1$-$C_6$)alkoxy-carbonyl; $-ZCO_2R'$ group; -CONRR' group; -NRR' group; -NRCOR' group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl or amino (-NRR');

with the proviso that one of A or B is an unsubstituted or substituted five-membered heterocycle as defined above where R and R' are hydrogen or ($C_1$-$C_6$)alkyl; Z is ($C_1$-$C_6$)alkyl; "amino" means NRR'; or an agronomically acceptable salt thereof.

2. A compound according to claim 1 wherein
   X and X' are O or S;
   $R^1$ is branched ($C_3$-$C_8$)alkyl; and
   A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; alkoxyalkyl having independently 1 to 4 carbon atoms in each alkyl group; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy or -NDD'; or phenoxy where the phenyl ring is unsubstituted or substituted with one or two of the same or different halo, nitro, ($C_1$-$C_4$)-alkyl, ($C_1$-$C_4$)alkoxy, carboxy, ($C_1$-$C_4$)alkoxy-carbonyl, ($C_1$-$C_4$)alkanoyloxy or -NDD'; or

   unsubstituted or substituted five-membered heterocycle selected from furyl, thienyl, triazolyl, pyrrolyl, and oxazolyl where the substituents can be one or two of the same or different halo; nitro; ($C_1$-$C_4$)alkyl; ($C_1$-$C_4$)alkoxy; -NDD'; or unsubstituted or substituted phenyl having one or two of the same or different halo, nitro, ($C_1$-$C_4$)alkyl, halo-($C_1$-$C_4$)-alkyl, ($C_1$-$C_4$)alkoxy, halo-($C_1$-$C_4$)alkoxy, carboxy or -NDD';

   one of A or B being an unsubstituted or substituted five-membered heterocycle as defined above where D and D' are hydrogen or ($C_1$-$C_4$)alkyl.

3. A compound according to claim 2 wherein
   X and X' are O;
   $R^1$ is branched ($C_4$-$C_7$)alkyl; and
   A and B are phenyl or substituted phenyl where the substituents can be from one to three of the same or different halo, nitro, ($C_1$-$C_4$)alkyl, ($C_1$-$C_4$)alkoxy or halo-($C_1$-$C_4$)alkyl; or

   unsubstituted or substituted five-membered heterocycle selected from furyl, thienyl pyrrolyl and oxazolyl where the substituents can be one or two of the same or different halo, nitro, ($C_1$-$C_4$)alkyl, ($C_1$-$C_4$)alkoxy or halo-($C_1$-$C_4$)alkyl; and

   one of A or B being an unsubstituted or substituted five-membered heterocycle as defined above.

4. A compound according to claim 3 wherein
   X and X' are O;
   $R^1$ is t-butyl, neopentyl (2,2-dimethylpropyl) or 1,2,2-trimethylpropyl; and
   A and B are phenyl or substituted phenyl where the substituents can be one or two of the same or different chloro, fluoro, bromo, iodo, nitro, methyl, ethyl, methoxy or trifluoromethyl; or unsubstituted furyl or thienyl or an unsubstituted or substituted pyrrolyl where the substituent can be ($C_1$-$C_4$)alkyl,

   one of A or B being an unsubstituted or substituted five-membered heterocycle as defined above.

5. The insecticidal compounds according to claim 1,
   N'-t-butyl-N-benzoyl-N'-(2-thiophenecarbonyl)hydrazine
   N'-t-butyl-N-(3-thiophenecarbonyl)-N'-benzoylhydrazine and
   N'-t-butyl-N-(3-furoyl)-N'-benzoylhydrazine.

6. An insecticidal composition comprising agronomically acceptable diluent carrier and an insecticidal compound according to any of claims 1 to 5.

7. A composition according to claim 5 wherein said compound is present at from 0.001 to about 90%, preferably 0.01 to about 75%, by weight of the composition.

8. An insecticidal composition according to claim 6 or 7 (a) containing a dispersing agent, said composition being in the form of a wettable powder or (b) containing a liquid agronomically acceptable

carrier and a dispersing agent, said composition being in the form of a flowable, or (c) in the form of a dust or (d) containing a binding agent, said composition being in the form of a granule or (e) containing an attractant agent, said composition being in the form of a bait or (f) containing an emulsifying agent, said composition being in the form of an emulsifiable concentrate.

**9.** A method of controlling insects which comprises contacting said insects with an insecticidal compound according to any of claims 1 to 5 optionally in the form of a composition according to any of claims 6 to 8.

**10.** A method according to claim 9 wherein said compound or composition is applied to growing plants or to the growth medium where the plants are growing or to be grown at from 10 grams to about 10 kilograms of active compound per hectare, preferably 100 grams to 5 kilograms per hectare.

**11.** A method according to claim 10 wherein said insects are from the order Lepidoptera.

**Claims for the following Contracting State : AT**

**1.** An insecticidal composition comprising an insecticidally active compound which is a substituted diacylhydrazine of the formula

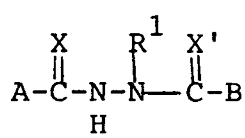

wherein

| | |
|---|---|
| X and X' | are the same or different O, S or NR; |
| $R^1$ is | unsubstituted ($C_3$-$C_{10}$) branched alkyl or a ($C_1$-$C_4$) straight chain alkyl substituted with one or two of the same or different ($C_3$-$C_6$)cycloalkyl; and |
| A and B | are unsubstituted or substituted phenyl where the substituents can be from one to five of the same or different halo; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; alkoxyalkyl having independently 1 to 6 carbon atoms in each akyl group; alkoxyalkoxy having independently 1 to 6 carbon atoms in each alkyl group; $-OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)-alkylthio; carboxy; $-ZCO_2R'$ group; -COR'; halo-($C_1$-$C_6$)alkyl-carbonyl; cyano-($C_1$-$C_6$)-alkyl-carbonyl; nitro-($C_1$-$C_6$)alkyl-carbonyl; ($C_1$-$C_6$)alkoxy-carbonyl; halo-($C_1$-$C_6$)alkoxy-carbonyl- -OCOR group; -NRR' group; amino substituted with hydroxy, ($C_1$-$C_4$)alkoxy or ($C_1$-$C_4$)alkylthio groups; -CONRR' group; -OCONRR' group; -NRCOR' group; $-NRCO_2R'$ group; -OCONRCOR' group; sulfhydryl; ($C_1$-$C_6$)alkylthio; halo-($C_1$-$C_6$)alkylthio; -NRCSR' group; -SCOR group;<br>or unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$c_4$)alkoxy-carbonyl, ($C_1$-$C_4$) alkanoyloxy or amino;<br>or phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$) alkoxy-carbonyl, ($C_1$-$C_4$)alkanoyloxy or amino;<br>or benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;<br>or phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;<br>or phenylthio where the phenyl ring is unsubstituted or substituted with one to three |

of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy or amino;

or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substitutents can be from one to three of the same or different halo; nitro; hydroxy; $(C_1-C_6)$alkyl; $(C_1-C_6)$alkoxy; carboxy; $(C_1-C_6)$alkoxy-carbonyl; $-ZCO_2R'$ group; -CONRR' group; -NRR' group; -NRCOR' group; $(C_1-C_6)$alkylthio; or unsubstituted or substituted phenyl having one to three of the same or different halo, nitro, $(C_1-C_6)$alkyl, halo-$(C_1-C_6)$alkyl, $(C_1-C_6)$alkoxy, halo-$(C_1-C_6)$alkoxy, carboxy, $(C_1-C_4)$alkoxy-carbonyl or amino (-NRR');

with the proviso that one of A or B is an unsubstituted or substituted five-membered heterocycle as defined above where R and R' are hydrogen or $(C_1-C_6)$alkyl; Z is $(C_1-C_6)$alkyl; "amino" means NRR'; or an

agronomically acceptable salt thereof.

**2.** A composition according to claim 1 wherein

X and X' are O or S;

$R^1$ is branched $(C_3-C_8)$alkyl; and

A and B are unsubstituted or substituted phenyl having one to three of the same or different halo; nitro; cyano; $(C_1-C_4)$alkyl; halo-$(C_1-C_4)$alkyl; cyano-$(C_1-C_4)$alkyl; $(C_1-C_4)$alkoxy; alkoxyalkyl having independently 1 to 4 carbon atoms in each alkyl group; -COD; $(C_1-C_4)$alkoxy-carbonyl; $(C_1-C_4)$alkanoyloxy; unsubstituted or substituted phenyl having one or two of the same or different halo, nitro, $(C_1-C_4)$alkyl, $(C_1-C_4)$alkoxy, carboxy, $(C_1-C_4)$-alkoxy-carbonyl, $(C_1-C_4)$alkanoyloxy or -NDD'; or phenoxy where the phenyl ring is unsubstituted or substituted with one or two of the same or different halo, nitro, $(C_1-C_4)$-alkyl, $(C_1-C_4)$alkoxy, carboxy, $(C_1-C_4)$alkoxy-carbonyl, $(C_1-C_4)$alkanoyloxy or -NDD'; or

unsubstituted or substituted five-membered heterocycle selected from furyl, thienyl, triazolyl, pyrrolyl, and oxazolyl where the substituents can be one or two of the same or different halo; nitro; $(C_1-C_4)$alkyl; $(C_1-C_4)$alkoxy; -NDD'; or unsubstituted or substituted phenyl having one or two of the same or different halo, nitro, $(C_1-C_4)$alkyl, halo-$(C_1-C_4)$-alkyl, $(C_1-C_4)$alkoxy, halo-$(C_1-C_4)$alkoxy, carboxy or -NDD';

one of A or B being an unsubstituted or substituted five-membered heterocycle as defined above where D and D' are hydrogen or $(C_1-C_4)$alkyl.

**3.** A composition according to claim 2 wherein

X and X' are O;

$R^1$ is branched $(C_4-C_7)$alkyl; and

A and B are phenyl or substituted phenyl where the substituents can be from one to three of the same or different halo, nitro, $(C_1-C_4)$alkyl, $(C_1-C_4)$alkoxy or halo-$(C_1-C_4)$alkyl; or

unsubstituted or substituted five-membered heterocycle selected from furyl, thienyl pyrrolyl and oxazolyl where the substituents can be one or two of the same or different halo, nitro, $(C_1-C_4)$alkyl, $(C_1-C_4)$alkoxy or halo-$(C_1-C_4)$alkyl; and

one of A or B being an unsubstituted or substituted five-membered heterocycle as defined above.

**4.** A composition according to claim 3 wherein

X and X' are O;

$R^1$ is t-butyl, neopentyl (2,2-dimethylpropyl) or 1,2,2-trimethylpropyl; and

A and B are phenyl or substituted phenyl where the substituents can be one or two of the same

or different chloro, fluoro, bromo, iodo, nitro, methyl, ethyl, methoxy or trifluoromethyl; or unsubstituted furyl or thienyl or an unsubstituted or substituted pyrrolyl where the substituent can be $(C_1\text{-}C_4)$alkyl,

one of A or B being an unsubstituted or substituted five-membered heterocycle as defined above.

**5.** A composition according to claim 1 wherein the insecticidally active compound comprises one or more of,
N'-t-butyl-N-benzoyl-N'-(2-thiophenecarbonyl)hydrazine
N'-t-butyl-N-(3-thiophenecarbonyl)-N'-benzoylhydrazine and
N'-t-butyl-N-(3-furoyl)-N'-benzoylhydrazine.

**6.** A composition according to any preceding claim wherein said compound is present at from 0.001 to about 90%, preferably 0.01 to about 75%, by weight of the composition.

**7.** An insecticidal composition according to any preceding claim (a) containing a dispersing agent, said composition being in the form of a wettable powder or (b) containing a liquid agronomically acceptable carrier and a dispersing agent, said composition being in the form of a flowable, or (c) in the form of a dust or (d) containing a binding agent, said composition being in the form of a granule or (e) containing an attractant agent, said composition being in the form of a bait or (f) containing an emulsifying agent, said composition being in the form of an emulsifiable concentrate.

**8.** A method of controlling insects which comprises contacting said insects with an insecticidal compound as defined in any of claims 1 to 5 optionally in a composition according to any preceding claim.

**9.** A method according to claim 8 wherein said compound or composition is applied to growing plants or to the growth medium where the plants are growing or to be grown at from 10 grams to about 10 kilograms of active compound per hectare, preferably 100 grams to 5 kilograms per hectare.

**10.** A method according to claim 9 wherein said insects are from the order Lepidoptera.

**Claims for the following Contracting State : ES**

**1.** The use of an insecticidally active compound which is a substituted diacylhydrazine of the formula

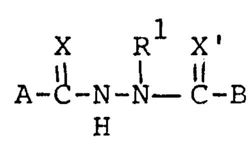

wherein

X and X' are the same or different O, S or NR;

$R^1$ is unsubstituted $(C_3\text{-}C_{10})$ branched alkyl or a $(C_1\text{-}C_4)$ straight chain alkyl substituted with one or two of the same or different $(C_3\text{-}C_6)$cycloalkyl; and

A and B are unsubstituted or substituted phenyl where the substituents can be from one to five of the same or different halo; nitro; cyano; hydroxy; $(C_1\text{-}C_6)$alkyl; halo-$(C_1\text{-}C_6)$alkyl; cyano-$(C_1\text{-}C_6)$alkyl; $(C_1\text{-}C_6)$alkoxy; halo-$(C_1\text{-}C_6)$alkoxy; alkoxyalkyl having independently 1 to 6 carbon atoms in each akyl group; alkoxyalkoxy having independently 1 to 6 carbon atoms in each alkyl group; $-OCO_2R$ group; $(C_2\text{-}C_6)$alkenyl optionally substituted with halo, cyano, $(C_1\text{-}C_4)$alkyl, $(C_1\text{-}C_4)$alkoxy, halo-$(C_1\text{-}C_4)$alkoxy or $(C_1\text{-}C_4)$alkylthio; $(C_2\text{-}C_6)$alkenyl-carbonyl; $(C_2\text{-}C_6)$alkadienyl; $(C_2\text{-}C_6)$alkynyl optionally substituted with halo, cyano, nitro, hydroxy, $(C_1\text{-}C_4)$alkyl, $(C_1\text{-}C_4)$alkoxy, halo-$(C_1\text{-}C_4)$alkoxy or $(C_1\text{-}C_4)$-alkylthio; carboxy; $-ZCO_2R'$ group; -COR'; halo-$(C_1\text{-}C_6)$alkyl-carbonyl; cyano-$(C_1\text{-}C_6)$-alkyl-carbonyl; nitro-$(C_1\text{-}C_6)$alkyl-carbonyl; $(C_1\text{-}C_6)$alkoxy-carbonyl; halo-$(C_1\text{-}C_6)$alkoxy-carbonyl- -OCOR group; -NRR' group; amino substituted with hydroxy, $(C_1\text{-}C_4)$alkoxy or

($C_1$-$C_4$)alkylthio groups; -CONRR' group; -OCONRR' group; -NRCOR' group; -$NRCO_2R'$ group; -OCONRCOR' group; sulfhydryl; ($C_1$-$C_6$)alkylthio; halo-($C_1$-$C_6$)alkylthio; -NRCSR' group; -SCOR group;

or unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino;

or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted five-membered heterocycle selected from furyl, thienyl, triazolyl, pyrrolyl, isopyrrolyl, pyrazolyl, isoimidazolyl, thiazolyl, isothiazolyl, oxazolyl and isooxazolyl where the substitutents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; -$ZCO_2R'$ group; -CONRR' group; -NRR' group; -NRCOR' group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl or amino (-NRR');

with the proviso that one of A or B is an unsubstituted or substituted five-membered heterocycle as defined above where R and R' are hydrogen or ($C_1$-$C_6$)alkyl; Z is ($C_1$-$C_6$)alkyl; "amino" means NRR'; or an agronomically acceptable salt thereof.

**2.** The use according to claim 1 wherein

X and X' are O or S;

$R^1$ is branched ($C_3$-$C_8$)alkyl; and

A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; alkoxyalkyl having independently 1 to 4 carbon atoms in each alkyl group; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy or -NDD'; or phenoxy where the phenyl ring is unsubstituted or substituted with one or two of the same or different halo, nitro, ($C_1$-$C_4$)-alkyl, ($C_1$-$C_4$)alkoxy, carboxy, ($C_1$-$C_4$)alkoxy-carbonyl, ($C_1$-$C_4$)alkanoyloxy or -NDD'; or

unsubstituted or substituted five-membered heterocycle selected from furyl, thienyl, triazolyl, pyrrolyl, and oxazolyl where the substituents can be one or two of the same or different halo; nitro; ($C_1$-$C_4$)alkyl; ($C_1$-$C_4$)alkoxy; -NDD'; or unsubstituted or substituted phenyl having one or two of the same or different halo, nitro, ($C_1$-$C_4$)alkyl, halo-($C_1$-$C_4$)-alkyl, ($C_1$-$C_4$)alkoxy, halo-($C_1$-$C_4$)alkoxy, carboxy or -NDD';

one of A or B being an unsubstituted or substituted five-membered heterocycle as defined above where D and D' are hydrogen or ($C_1$-$C_4$)alkyl.

**3.** The use according to claim 2 wherein

X and X' are O;
$R^1$ is branched ($C_4$-$C_7$)alkyl; and
A and B are phenyl or substituted phenyl where the substituents can be from one to three of the same or different halo, nitro, ($C_1$-$C_4$)alkyl, ($C_1$-$C_4$)alkoxy or halo-($C_1$-$C_4$)alkyl; or

unsubstituted or substituted five-membered heterocycle selected from furyl, thienyl pyrrolyl and oxazolyl where the substituents can be one or two of the same or different halo, nitro, ($C_1$-$C_4$)alkyl, ($C_1$-$C_4$)alkoxy or halo-($C_1$-$C_4$)alkyl; and

one of A or B being an unsubstituted or substituted five-membered heterocycle as defined above.

**4.** The use according to claim 3 wherein
X and X' are O;
$R^1$ is t-butyl, neopentyl (2,2-dimethylpropyl) or 1,2,2-trimethylpropyl; and
A and B are phenyl or substituted phenyl where the substituents can be one or two of the same or different chloro, fluoro, bromo, iodo, nitro, methyl, ethyl, methoxy or trifluoromethyl; or
unsubstituted furyl or thienyl or an unsubstituted or substituted pyrrolyl where the substituent can be ($C_1$-$C_4$)alkyl,

one of A or B being an unsubstituted or substituted five-membered heterocycle as defined above.

**5.** The use according to claim 1 wherein the insecticidal compound comprises one or more of
N'-t-butyl-N-benzoyl-N'-(2-thiophenecarbonyl)hydrazine
N'-t-butyl-N-(3-thiophenecarbonyl)-N'-benzoylhydrazine and
N'-t-butyl-N-(3-furoyl)-N'-benzoylhydrazine.

**6.** The use of an insecticidal compound as defined in any of claims 1 to 5 optionally in a composition also containing agronomically acceptable diluent or carrier, for controlling insects by contacting said insects with an effective amount of the compound or composition.

**7.** The use according to any preceding claim wherein said compound is present in said composition at from 0.001 to about 90%, preferably 0.01 to about 75%, by weight of the composition.

**8.** The use according to any preceding claim wherein said insecticidal composition contains a dispersing agent, said composition being in the form of a wettable powder or (b) contains a liquid agronomically acceptable carrier and a dispersing agent, said composition being in the form of a flowable, or (c) is in the form of a dust or (d) contains a binding agent, said composition being in the form of a granule or (e) contains an attractant agent, said composition being in the form of a bait or (f) containing an emulsifying agent, said composition being in the form of an emulsifiable concentrate.

**9.** The use according to any of claims 6 to 8 wherein said compound or composition is applied to growing plants or to the growth medium where the plants are growing or to be grown at from 10 grams to about 10 kilograms of active compound per hectare, preferably 100 grams to 5 kilograms per hectare.

**10.** The use according to claim 9 wherein said insects are from the order Lepidoptera.

**Patentansprüche**
**Patentansprüche für folgende Vertragsstaaten : BE, CH, DE, FR, GB, GR, IT, LI, LU, NL, SE**

**1.** Insektizid-aktive Verbindung, nämlich ein substituiertes Diacylhydrazin der Formel:

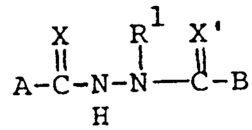

worin

X und X' gleich oder verschieden voneinander O, S oder NR sind,

$R^1$ ein unsubstituiertes, verzweigtes ($C_3$-$C_{10}$)Alkyl oder ein geradkettiges, mit ein oder zwei der gleichen oder verschiedenen von ($C_3$-$C_6$)Cycloalkyl substituiertes ($C_1$-$C_4$)Alkyl ist, und

A und B ein unsubstituiertes oder substituiertes Phenyl, wo die Substituenten ein bis fünf der gleichen oder verschiedenen von Halogen, Nitro, Cyano, Hydroxy, ($C_1$-$C_6$)Alkyl, Halogen($C_1$-$C_6$)alkyl, Cyano($C_1$-$C_6$)alkyl, ($C_1$-$C_6$)Alkoxy, Halogen($C_1$-$C_6$)alkoxy, Alkoxyalkyl mit, unabhängig davon, 1 bis 6 C-Atomen in jedem Alkyl-Rest, Alkoxyalkoxy mit, unabhängig davon, 1 bis 6 C-Atomen in jedem Alkyl-Rest, einem $-OCO_2R$-Rest, ($C_2$-$C_6$)-Alkenyl, gegebenenfalls substituiert mit Halogen, Cyano, ($C_1$-$C_4$)Alkyl, ($C_1$-$C_4$)-Alkoxy, Halogen ($C_1$-$C_4$)alkoxy oder ($C_1$-$C_4$) Alkylthio, ($C_2$-$C_6$)Alkenylcarbonyl, ($C_2$-$C_6$)-Alkadienyl, ($C_2$-$C_6$)Alkynyl, gegebenenfalls substituiert mit Halogen, Cyano, Nitro, Hydroxy, ($C_1$-$C_4$)- Alkyl, ($C_1$-$C_4$)Alkoxy, Halogen($C_1$-$C_4$)- alkoxy oder ($C_1$-$C_4$)Alkylthio, Carboxy, einem $-ZCO_2R'$-Rest, einem -COR'-Rest, Halogen($C_1$-$C_6$)alkylcarbonyl, Cyano($C_1$-$C_6$)alkylcarbonyl, Nitro($C_1$-$C_6$)alkylcarbonyl, ($C_1$-$C_6$)Alkoxycarbonyl, Halogen-($C_1$-$C_6$)alkoxycarbonyl, einem -OCOR-Rest, einem -NRR'-Rest, Amino, substituiert mit Hydroxy, ($C_1$-$C_4$)Alkoxy- oder ($C_1$-$C_4$)Alkylthio-Resten, einem -CONRR'-Rest, einem -OCONRR'-Rest, einem -NRCOR'-Rest, einem $-NRCO_2R'$-Rest, einem -OCONRCOR'-Rest, Sulfhydryl, ($C_1$-$C_6$)Alkyl- thio, Halogen($C_1$-$C_6$)alkylthio, einem -NRCSR'-Rest, einem -SCOR-Rest, sein können,

ein unsubstituiertes oder substituiertes Phenyl mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino,

Phenoxy, wo der Phenylring unsubstituiert oder mit ein bis drei der gleichen oder verschiedenen von Halogen, Cyano, Nitro, Hydroxy,

($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino, substituiert ist,

Benzoyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino substituiert ist,

Phenoxycarbonyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, oder Amino substituiert ist,

Phenylthio, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino substituiert ist, sind,

oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterozyklischen Ring auszubilden, oder

ein unsubstituierter oder substituierter, aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl ausgewählter 5-gliedriger Heterozyklus, wo die Substituenten ein bis drei der gleichen oder verschiedenen von Halogen, Nitro, Hydroxy, $(C_1-C_6)$Alkyl, $(C_1-C_6)$- Alkoxy, Carboxy, $(C_1-C_6)$Alkoxycarbonyl, einem $-ZCO_2R'$-Rest einem -CONRR'-Rest, einem -NRR'-Rest, einem -NRCOR'-Rest oder $(C_1-C_6)$Alkylthio sein können, oder

ein unsubstituiertes oder substituiertes Phenyl, wo die Substituenten ein bis drei der gleichen oder verschiedenen von Halogen, Nitro, $(C_1-C_6)$Alkyl, Halogen$(C_1-C_6)$alkyl, $(C_1-C_6)$Alkoxy, Halogen$(C_1-C_6)$alkoxy, Carboxy, $(C_1-C_4)$Alkoxycarbonyl oder Amino(-NRR') sein können, sind

mit der Maßgabe, daß A oder B ein unsubstituierter oder wie vorstehend angegebener substituierter 5-gliedriger Heterozyklus ist, wo R und R' Wasserstoff oder $(C_1-C_6)$Alkyl sind, Z $(C_1-C_6)$Alkyl ist, "Amino" NRR' bedeutet,

oder ein landwirtschafltich verträgliches Salz davon.

**2.** Verbindung nach Anspruch 1, worin

X und x' O oder S sind,

$R^1$ ein verzweigtes $(C_3-C_8)$Alkyl ist, und

A und B ein unsubstituiertes oder substituiertes Phenyl, wo die Substituenten ein bis drei der gleichen oder verschiedenen von Halogen, Nitro, Cyano, $(C_1-C_4)$Alkyl, Halogen$(C_1-C_4)$-alkyl, Cyano$(C_1-C_4)$alkyl, $(C_1-C_4)$Alkoxy, Alkoxyalkyl mit, unabhängig davon, 1 bis 4 C-Atomen in jedem Alkyl-Rest, einem -COD-Rest, $(C_1-C_4)$Alkoxycarbonyl, $(C_1-C_4)$-Alkanoyloxy sein können,

ein unsubstituiertes oder substituiertes Phenyl, wo die Substituenten ein oder zwei der gleichen oder verschiedenen von Halogen, Nitro, $(C_1-C_4)$Alkyl, $(C_1-C_4)$Alkoxy, Carboxy, $(C_1-C_4)$Alkoxycarbonyl, $(C_1-C_4)$Alkanoyloxy oder -NDD' sein können,

Phenoxy, wo der Phenylring unsubstituiert oder mit ein oder zwei der gleichen oder verschiedenen von Halogen, Nitro, $(C_1-C_4)$Alkyl, $(C_1-C_4)$Alkoxy, Carboxy, $(C_1-C_4)$-Alkoxycarbonyl, $(C_1-C_4)$Alkanoyloxy oder -NDD' substituiert ist,

ein unsubstituierter oder substituierter, aus Furyl, Thienyl, Triazolyl, Pyrrolyl und Oxazolyl ausgewählter 5-gliedriger Heterozyklus, wo die Substituenten ein oder zwei der gleichen oder verschiedenen von Halogen, Nitro, $(C_1-C_4)$Alkyl, $(C_1-C_4)$Alkoxy oder -NDD' sein können, oder

ein unsubstituiertes oder substituiertes Phenyl, wo die Substituenten ein oder zwei der gleichen oder verschiedenen von Halogen, Nitro, $(C_1-C_4)$Alkyl, Halogen$(C_1-C_4)$alkyl, $(C_1-C_4)$Alkoxy, Halogen$(C_1-C_4)$alkoxy, Carboxy oder -NDD' sein können, sind,

wobei A oder B ein unsubstituierter oder wie vorstehend angegebener substituierter 5-gliedriger Heterozyklus ist, wo D und D' Wasserstoff oder $(C_1-C_4)$Alkyl sind.

**3.** Verbindung nach Anspruch 2, worin

X und X' O sind,

$R^1$ ein verzweigtes $(C_4-C_7)$Alkyl ist, und

A und B ein unsubstituiertes oder substituiertes Phenyl, wo die Substituenten ein bis drei der gleichen oder verschiedenen von Halogen, Nitro, $(C_1-C_4)$Alkyl, Halogen$(C_1-C_4)$alkyl oder $(C_1-C_4)$Alkoxy sein können,

oder ein unsubstituierter oder substituierter aus Furyl, Thienyl, Pyrrolyl und Oxazolyl ausgewählter Heterozyklus, wo die Substituenten ein oder zwei der gleichen oder verschiedenen von Halogen, Nitro, $(C_1-C_4)$Alkyl, $(C_1-C_4)$Alkoxy oder Halogen$(C_1-C_4)$-alkyl sein können, sind,

wobei A oder B ein unsubstituierter oder wie vorstehend angegebener substituierter, 5-gliedriger Heterozyklus ist.

**4.** Verbindung nach Anspruch 3, worin

X und X' 0 sind,

$R^1$ t-Butyl, Neopentyl(2,2-dimethylpropyl) oder 1,2,2,-Trimethylpropyl ist, und

A und B ein unsubstituiertes oder substituiertes Phenyl, wo die Substituenten ein oder zwei der gleichen oder verschiedenen von Chlor, Fluor, Brom, Jod, Nitro, Methyl, Ethyl, Methoxy oder Trifluormethyl sein können, oder ein unsubstituiertes Furyl oder Thienyl oder ein unsubstituiertes oder substituiertes Pyrrolyl, wo der Substituent $(C_1-C_4)$Alkyl sein kann, sind,

wobei A oder B ein unsubstituierter oder wie vorstehend angegebener substituierter, 5-gliedriger Heterozyklus ist.

**5.** Insektizide Verbindungen nach Anspruch 1, nämlich N'-t-Butyl-N-benzoyl-N'-(2-thiophencarbonyl)-hydrazin, N'-t-Butyl-N-(3-thiophencarbonyl)-N'-benzoylhydrazin und N'-t-Butyl-N-(3-furoyl)-N'-benzoylhydrazin.

**6.** Insektizides Mittel, umfassend einen landwirtschaftlich verträglichen Verdünnungsmittel-Träger und eine insektizide Verbindung nach einem der Ansprüche 1 bis 5.

**7.** Mittel nach Anspruch 5, worin die vorliegende Verbindung 0,001 bis etwa 90 Gew.-%, vorzugsweise 0,01 bis etwa 75 Gew.-% des Mittels beträgt.

**8.** Insektizides Mittel nach Anspruch 6 oder 7, enthaltend (a) ein Dispersionsmittel, wobei das Mittel in Form eines benetzbaren Pulvers vorliegt, (b) einen flüssigen landwirtschaftlich verträglichen Träger und ein Dispersionsmittel, wobei das Mittel in fließbarer Form oder (c) in Form von Staub vorliegt, (d) ein Bindemittel, wobei das Mittel in Form von Granulat vorliegt, (e) einen Lockstoff, wobei das Mittel in Form eines Köders vorliegt,

oder (f) ein Emulgiermittel, wobei das Mittel in Form eines emulgierbaren Konzentrats vorliegt.

**9.** Verfahren zur Kontrolle von Insekten, bei dem man die Insekten mit einer insektiziden Verbindung nach einem der Ansprüche 1 bis 5, gegebenenfalls in Form eines Mittels nach einem der Ansprüche 6 bis 8 in Kontakt bringt.

**10.** Verfahren nach Anspruch 9, worin die Verbindung oder das Mittel wachsenden Pflanzen oder dem Wachstumsmedium, in dem Pflanzen wachsen oder wachsen sollen, mit einer Rate von 10 g bis etwa 10 kg der wirksamen Verbindung pro Hektar, vorzugsweise 100 g bis 5 kg pro Hektar verabreicht wird.

**11.** Verfahren nach Anspruch 10, worin die Insekten der Ordnung Lepidoptera angehören.

**Patentansprüche für folgenden Vertragsstaat : AT**

**1.** Insektizides Mittel, enthaltend eine Insektizid-aktive Verbindung, nämlich ein substituiertes Diacylhydrazin der Formel

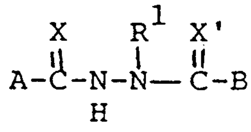

worin

X und X' gleich oder verschieden voneinander O, S oder NR sind,

$R^1$ ein unsubstituiertes, verzweigtes $(C_3-C_{10})$Alkyl oder ein geradkettiges, mit ein oder zwei der gleichen oder verschiedenen von $(C_3-C_6)$Cycloalkyl substituiertes $(C_1-C_4)$Alkyl ist,

und

A und B ein unsubstituiertes oder substituiertes Phenyl, wo die Substituenten ein bis fünf der gleichen oder verschiedenen von Halogen, Nitro, Cyano, Hydroxy, $(C_1-C_6)$Alkyl, Halogen$(C_1-C_6)$alkyl, Cyano$(C_1-C_6)$alkyl, $(C_1-C_6)$Alkoxy, Halogen$(C_1-C_6)$alkoxy, Alkoxyalkyl mit, unabhängig davon, 1 bis 6 C-Atomen in jedem Alkyl-Rest, Alkoxvalkoxy mit, unabhängig davon, 1 bis 6 C-Atomen in jedem Alkyl-Rest, einem $-OCO_2R$-Rest, $(C_2-C_6)$-Alkenyl, gegebenenfalls substituiert mit Halogen, Cyano, $(C_1-C_4)$Alkyl, $(C_1-C_4)$-Alkoxy, Halogen $(C_1-C_4)$alkoxy oder $(C_1-C_4)$ Alkylthio, $(C_2-C_6)$Alkenylcarbonyl, $(C_2-C_6)$-Alkadienyl, $(C_2-C_6)$Alkynyl, gegebenenfalls substituiert mit Halogen, Cyano, Nitro, Hydroxy, $(C_1-C_4)$Alkyl, $(C_1-C_4)$Alkoxy, Halogen$(C_1-C_4)$alkoxy oder $(C_1-C_4)$Alkylthio, Carboxy, einem $-ZCO_2R'$-Rest, einem -COR'-Rest, Halogen- $(C_1-C_6)$alkylcarbonyl, Cyano-$(C_1-C_6)$- alkylcarbonyl, Nitro$(C_1-C_6)$alkylcarbonyl, $(C_1-C_6)$Alkoxycarbonyl, Halogen$(C_1-C_6)$- alkoxycarbonyl, einem -OCOR-Rest, einem -NRR'-Rest, Amino, substituiert mit Hydroxy, $(C_1-C_4)$Alkoxy- oder $(C_1-C_4)$Alkylthio- Resten, einem -CONRR'-Rest, einem -OCONRR'-Rest, einem -NRCOR'-Rest, einem $-NRCO_2R'$-Rest, einem -OCONRCOR'-Rest, Sulfhydryl, $(C_1-C_6)$Alkylthio, Halogen$(C_1-C_6)$alkylthio, einem -NRCSR'-Rest, einem -SCOR-Rest, sein können,

ein unsubstituiertes oder substituiertes Phenyl mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$Alkoxycarbonyl, $(C_1-C_4)$Alkanoyloxy oder Amino,

Phenoxy, wo der Phenylring unsubstituiert oder mit ein bis drei der gleichen oder verschiedenen von Halogen, Cyano, Nitro, Hydroxy,

$(C_1-C_4)$Alkyl, $(C_1-C_4)$Alkoxy, Carboxy, $(C_1-C_4)$Alkoxycarbonyl, $(C_1-C_4)$Alkanoyloxy oder Amino, substituiert ist,

Benzoyl, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$Alkoxycarbonyl, $(C_1-C_4)$Alkanoyloxy oder Amino substituiert ist,

Phenoxycarbonyl, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$Alkoxycarbonyl, $(C_1-C_4)$Alkanoyloxy, oder Amino substituiert ist,

Phenylthio, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$Alkoxycarbonyl, $(C_1-C_4)$Alkanoyloxy oder Amino substituiert ist, sind,

oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterozyklischen Ring auszubilden, oder

ein unsubstituierter oder substituierter, aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl ausgewählter 5-gliedriger Heterozyklus, wo die Substituenten ein bis drei der gleichen oder verschiedenen von Halogen, Nitro, Hydroxy, $(C_1-C_6)$Alkyl, $(C_1-C_6)$- Alkoxy, Carboxy, $(C_1-C_6)$Alkoxycarbonyl, einem $-ZCO_2R'$-Rest einem -CONRR'-Rest, einem -NRR'-Rest, einem -NRCOR'-Rest oder $(C_1-C_6)$Alkylthio sein können, oder

ein unsubstituiertes oder substituiertes Phenyl, wo die Substituenten ein bis drei der gleichen oder verschiedenen von Halogen, Nitro, $(C_1-C_6)$Alkyl, Halogen$(C_1-C_6)$alkyl, $(C_1-C_6)$Alkoxy, Halogen$(C_1-C_6)$alkoxy, Carboxy, $(C_1-C_4)$Alkoxycarbonyl oder Amino(-NRR') sein können, sind

mit der Maßgabe, daß A oder B ein unsubstituierter oder wie vorstehend angegebener

substituierter 5-gliedriger Heterozyklus ist, wo R und R' Wasserstoff oder $(C_1\text{-}C_6)$Alkyl sind, Z $(C_1\text{-}C_6)$Alkyl ist, "Amino" NRR' bedeutet,

oder ein landwirtschafltich verträgliches Salz davon.

**2.** Verbindung nach Anspruch 1, worin

X und X' O oder S sind,

$R^1$ ein verzweigtes $(C_3\text{-}C_8)$Alkyl ist, und

A und B ein unsubstituiertes oder substituiertes Phenyl, wo die Substituenten ein bis drei der gleichen oder verschiedenen von Halogen, Nitro, Cyano, $(C_1\text{-}C_4)$Alkyl, Halogen$(C_1\text{-}C_4)$-alkyl, Cyano$(C_1\text{-}C_4)$alkyl, $(C_1\text{-}C_4)$Alkoxy, Alkoxyalkyl mit, unabhängig davon, 1 bis 4 C-Atomen in jedem Alkyl-Rest, einem -COD-Rest, $(C_1\text{-}C_4)$Alkoxycarbonyl, $(C_1\text{-}C_4)$-Alkanoyloxy sein können,

ein unsubstituiertes oder substituiertes Phenyl, wo die Substituenten ein oder zwei der gleichen oder verschiedenen von Halogen, Nitro, $(C_1\text{-}C_4)$Alkyl, $(C_1\text{-}C_4)$Alkoxy, Carboxy, $(C_1\text{-}C_4)$Alkoxycarbonyl, $(C_1\text{-}C_4)$Alkanoyloxy oder -NDD' sein können,

Phenoxy, wo der Phenylring unsubstituiert oder mit ein oder zwei der gleichen oder verschiedenen von Halogen, Nitro, $(C_1\text{-}C_4)$Alkyl, $(C_1\text{-}C_4)$Alkoxy, Carboxy, $(C_1\text{-}C_4)$-Alkoxycarbonyl, $(C_1\text{-}C_4)$- Alkanoyloxy oder -NDD' substituiert ist,

ein unsubstituierter oder substituierter, aus Furyl, Thienyl, Triazolyl, Pyrrolyl und Oxazolyl ausgewählter 5-gliedriger Heterozyklus, wo die Substituenten ein oder zwei der gleichen oder verschiedenen von Halogen, Nitro, $(C_1\text{-}C_4)$Alkyl, $(C_1\text{-}C_4)$Alkoxy oder -NDD' sein können, oder

ein unsubstituiertes oder substituiertes Phenyl, wo die Substituenten ein oder zwei der gleichen oder verschiedenen von Halogen, Nitro, $(C_1\text{-}C_4)$Alkyl, Halogen$(C_1\text{-}C_4)$alkyl, $(C_1\text{-}C_4)$Alkoxy, Halogen$(C_1\text{-}C_4)$alkoxy, Carboxy oder -NDD' sein können, sind,

wobei A oder B ein unsubstituierter oder wie vorstehend angegebener substituierter 5-gliedriger Heterozyklus ist, wo D und D' Wasserstoff oder $(C_1\text{-}C_4)$Alkyl sind.

**3.** Verbindung nach Anspruch 2, worin

X und X' O sind,

$R^1$ ein verzweigtes $(C_4\text{-}C_7)$Alkyl ist, und

A und B ein unsubstituiertes oder substituiertes Phenyl, wo die Substituenten ein bis drei der gleichen oder verschiedenen von Halogen, Nitro, $(C_1\text{-}C_4)$Alkyl, Halogen$(C_1\text{-}C_4)$alkyl oder $(C_1\text{-}C_4)$Alkoxy sein können,

oder ein unsubstituierter oder substituierter aus Furyl, Thienyl, Pyrrolyl und Oxazolyl ausgewählter Heterozyklus, wo die Substituenten ein oder zwei der gleichen oder verschiedenen von Halogen, Nitro, $(C_1\text{-}C_4)$Alkyl, $(C_1\text{-}C_4)$Alkoxy oder Halogen$(C_1\text{-}C_4)$-alkyl sein können, sind,

wobei A oder B ein unsubstituierter oder wie vorstehend angegebener substituierter, 5-gliedriger Heterozyklus ist.

**4.** Verbindung nach Anspruch 3, worin

X und X' 0 sind,

$R^1$ t-Butyl, Neopentyl(2,2-dimethylpropyl) oder 1,2,2,-Trimethylpropyl ist, und

A und B ein unsubstituiertes oder substituiertes Phenyl, wo die Substituenten ein oder zwei der gleichen oder verschiedenen von Chlor, Fluor, Brom, Jod, Nitro, Methyl, Ethyl, Methoxy oder Trifluormethyl sein können, oder

ein unsubstituiertes Furyl oder Thienyl oder ein unsubstituiertes oder substituiertes Pyrrolyl, wo der Substituent $(C_1\text{-}C_4)$Alkyl sein kann, sind

wobei A oder B ein unsubstituierter oder wie vorstehend angegebener substituierter, 5-gliedriger Heterozyklus ist.

**5.** Mittel nach Anspruch 1, worin die Insektizid-aktive Verbindung eine oder mehrere der Verbindungen
N'-t-Butyl-N-benzoyl-N'-(2-thiophencarbonyl)hydrazin,
N'-t-Butyl-N-(3-thiophencarbonyl)-N'-benzoylhydrazin und
N'-t-Butyl-N-(3-furoyl)-N'-benzoylhydrazin umfaßt.

**6.** Mittel nach einem der vorhergehenden Ansprüche, worin die vorliegende Verbindung 0,001 bis etwa 90 Gew.-%, vorzugsweise 0,01 bis etwa 75 Gew.-% des Mittels beträgt.

**7.** Insektizides Mittel gemäß einem der vorhergehenden Ansprüche, enthaltend (a) ein Dispersionsmittel, wobei das Mittel in Form eines benetzbaren Pulvers vorliegt, (b) einen flüssigen landwirtschaftlich verträglichen Träger und ein Dispersionsmittel, wobei das Mittel in fließbarer Form oder (c) in Form von Staub vorliegt, (d) ein Bindemittel, wobei das Mittel in Form von Granulat vorliegt, (e) einen Lockstoff, wobei das Mittel in Form eines Köders vorliegt,

oder (f) ein Emulgiermittel, wobei das Mittel in Form eines emulgierbaren Konzentrats vorliegt.

**8.** Verfahren zur Kontrolle von Insekten, bei dem man die Insekten mit einer insektiziden Verbindung nach einem der Ansprüche 1 bis 5, gegebenenfalls in Form eines Mittels nach einem der vorhergehenden Ansprüche in Kontakt bringt.

**9.** Verfahren nach Anspruch 8, worin die Verbindung oder das Mittel wachsenden Pflanzen oder dem Wachstumsmedium, in dem Pflanzen wachsen oder wachsen sollen, mit einer Rate von 10 g bis etwa 10 kg der wirksamen Verbindung pro Hektar, vorzugsweise 100 g bis 5 kg pro Hektar verabreicht wird.

**10.** Verfahren nach Anspruch 9, worin die Insekten der Ordnung Lepidoptera angehören.

**Patentansprüche für folgenden Vertragsstaat : ES**

**1.** Verwendung einer Insektizid-aktiven Verbindung, nämlich eines substituierten Diacylhydrazins der Formel:

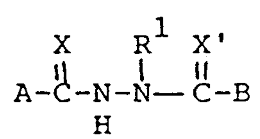

worin

X und X' gleich oder verschieden voneinander O, S oder NR sind,

$R^1$ ein unsubstituiertes, verzweigtes ($C_3$-$C_{10}$)Alkyl oder ein geradkettiges, mit ein oder zwei der gleichen oder verschiedenen von ($C_3$-$C_6$)Cycloalkyl substituiertes ($C_1$-$C_4$)Alkyl ist, und

A und B ein unsubstituiertes oder substituiertes Phenyl, wo die Substituenten ein bis fünf der gleichen oder verschiedenen von Halogen, Nitro, Cyano, Hydroxv, ($C_1$-$C_6$)Alkyl, Halogen($C_1$-$C_6$)alkyl, Cyano($C_1$-$C_6$)alkyl, ($C_1$-$C_6$)Alkoxy, Halogen($C_1$-$C_6$)alkoxy, Alkoxyalkyl mit, unabhängig davon, 1 bis 6 C-Atomen in jedem Alkyl-Rest, Alkoxyalkoxy mit, unabhängig davon, 1 bis 6 C-Atomen in jedem Alkyl-Rest, einem -$OCO_2R$-Rest, ($C_2$-$C_6$)-Alkenyl, gegebenenfalls substituiert mit Halogen, Cyano, ($C_1$-$C_4$)Alkyl, ($C_1$-$C_4$)-Alkoxy, Halogen ($C_1$-$C_4$)alkoxy oder ($C_1$-$C_4$) Alkylthio, ($C_2$-$C_6$)Alkenylcarbonyl, ($C_2$-$C_6$)-Alkadienyl, ($C_2$-$C_6$)Alkynyl, gegebenenfalls substituiert mit Halogen, Cyano, Nitro, Hydroxy, ($C_1$-$C_4$)Alkyl, ($C_1$-$C_4$)Alkoxy, Halogen($C_1$-$C_4$)alkoxy oder ($C_1$-$C_4$)Alkylthio, Carboxy, einem -$ZCO_2R'$-Rest, einem -COR'-Rest, Halogen- ($C_1$-$C_6$)alkylcarbonyl, Cyano-($C_1$-$C_6$)- alkylcarbonyl, Nitro($C_1$-$C_6$)alkylcarbonyl, ($C_1$-$C_6$)Alkoxycarbonyl, Halogen($C_1$-

$C_6$)- alkoxycarbonyl, einem -OCOR-Rest, einem -NRR'-Rest, Amino, substituiert mit Hydroxy, ($C_1$-$C_4$)Alkoxy- oder ($C_1$-$C_4$)Alkylthio- Resten, einem -CONRR'-Rest, einem -OCONRR'-Rest, einem -NRCOR'-Rest, einem -$NRCO_2R'$-Rest, einem -OCONRCOR'-Rest, Sulfhydryl, ($C_1$-$C_6$)Alkylthio, Halogen($C_1$-$C_6$)alkylthio, einem -NRCSR'-Rest, einem -SCOR-Rest, sein können,

ein unsubstituiertes oder substituiertes Phenyl mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino,

Phenoxy, wo der Phenylring unsubstituiert oder mit ein bis drei der gleichen oder verschiedenen von Halogen, Cyano, Nitro, Hydroxy,

($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino, substituiert ist,

Benzoyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino substituiert ist,

Phenoxycarbonyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, oder Amino substituiert ist,

Phenvlthio, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder Amino substituiert ist, sind,

oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterozyklischen Ring auszubilden, oder

ein unsubstituierter oder substituierter, aus Furyl, Thienyl, Triazolyl, Pyrrolyl, Isopyrrolyl, Pyrazolyl, Isoimidazolyl, Thiazolyl, Isothiazolyl, Oxazolyl und Isooxazolyl ausgewählter 5-gliedriger Heterozyklus, wo die Substituenten ein bis drei der gleichen oder verschiedenen von Halogen, Nitro, Hydroxy, ($C_1$-$C_6$)Alkyl, ($C_1$-$C_6$)Alkoxy, Carboxy, ($C_1$-$C_6$)Alkoxycarbonyl, einem -$ZCO_2R'$-Rest einem -CONRR'-Rest, einem -NRR'-Rest, einem -NRCOR'-Rest oder ($C_1$-$C_6$)Alkylthio sein können, oder

ein unsubstituiertes oder substituiertes Phenyl, wo die Substituenten ein bis drei der gleichen oder verschiedenen von Halogen, Nitro, ($C_1$-$C_6$)Alkyl, Halogen($C_1$-$C_6$)alkyl, ($C_1$-$C_6$)Alkoxy, Halogen($C_1$-$C_6$)alkoxy, Carboxy, ($C_1$-$C_4$)Alkoxycarbonyl oder Amino(-NRR') sein können, sind

mit der Maßgabe, daß A oder B ein unsubstituierter oder wie vorstehend angegebener substituierter 5-gliedriger Heterozyklus ist,

wo R und R' Wasserstoff oder ($C_1$-$C_6$)Alkyl sind, Z ($C_1$-$C_6$)Alkyl ist, "Amino" NRR' bedeutet,

oder ein landwirtschafltich verträgliches Salz davon.

**2.** Verwendung nach Anspruch 1, worin

| | |
|---|---|
| X und x' | O oder S sind, |
| $R^1$ ein | verzweigtes ($C_3$-$C_8$)Alkyl ist, und |
| A und B | ein unsubstituiertes oder substituiertes Phenyl, wo die Substituenten ein bis drei der gleichen oder verschiedenen von Halogen, Nitro, Cyano, ($C_1$-$C_4$)Alkyl, Halogen($C_1$-$C_4$)- |

alkyl, Cyano($C_1$-$C_4$)alkyl, ($C_1$-$C_4$)Alkoxy, Alkoxyalkyl mit, unabhängig davon, 1 bis 4 C-Atomen in jedem Alkyl-Rest, einem -COD-Rest, ($C_1$-$C_4$)Alkoxycarbonyl, ($C_1$-$C_4$)-Alkanoyloxy sein können,

ein unsubstituiertes oder substituiertes Phenyl, wo die Substituenten ein oder zwei der gleichen oder verschiedenen von Halogen, Nitro, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy oder -NDD' sein können,

Phenoxy, wo der Phenylring unsubstituiert oder mit ein oder zwei der gleichen oder verschiedenen von Halogen, Nitro, ($C_1$-$C_4$)Alkyl, ($C_1$-$C_4$)Alkoxy, Carboxy, ($C_1$-$C_4$)-Alkoxycarbonyl, ($C_1$-$C_4$)- Alkanoyloxy oder -NDD' substituiert ist,

ein unsubstituierter oder substituierter, aus Furyl, Thienyl, Triazolyl, Pyrrolyl und Oxazolyl ausgewählter 5-gliedriger Heterozyklus, wo die Substituenten ein oder zwei der gleichen oder verschiedenen von Halogen, Nitro, ($C_1$-$C_4$)Alkyl, ($C_1$-$C_4$)Alkoxy oder -NDD' sein können, oder

ein unsubstituiertes oder substituiertes Phenyl, wo die Substituenten ein oder zwei der gleichen oder verschiedenen von Halogen, Nitro, ($C_1$-$C_4$)Alkyl, Halogen($C_1$-$C_4$)alkyl, ($C_1$-$C_4$)Alkoxy, Halogen($C_1$-$C_4$)alkoxy, Carboxy oder -NDD' sein können, sind,

wobei A oder B ein unsubstituierter oder wie vorstehend angegebener substituierter 5-gliedriger Heterozyklus ist, wo D und D' Wasserstoff oder ($C_1$-$C_4$)Alkyl sind.

**3.** Verwendung nach Anspruch 2, worin

| | |
|---|---|
| X und X' | O sind, |
| $R^1$ ein | verzweigtes ($C_4$-$C_7$)Alkyl ist, und |
| A und B | ein unsubstituiertes oder substituiertes Phenyl, wo die Substituenten ein bis drei der gleichen oder verschiedenen von Halogen, Nitro, ($C_1$-$C_4$)Alkyl, Halogen($C_1$-$C_4$)alkyl oder ($C_1$-$C_4$)Alkoxy sein können, |

oder ein unsubstituierter oder substituierter aus Furyl, Thienyl, Pyrrolyl und Oxazolyl ausgewählter Heterozyklus, wo die Substituenten ein oder zwei der gleichen oder verschiedenen von Halogen, Nitro, ($C_1$-$C_4$)Alkyl, ($C_1$-$C_4$)Alkoxy oder Halogen($C_1$-$C_4$)-alkyl sein können, sind,

wobei A oder B ein unsubstituierter oder wie vorstehend angegebener substituierter, 5-gliedriger Heterozyklus ist.

**4.** Verwendung nach Anspruch 3, worin

| | |
|---|---|
| X und X' | 0 sind, |
| $R^1$ | t-Butyl, Neopentyl(2,2-dimethylpropyl) oder 1,2,2,-Trimethylpropyl ist, und |
| A und B | ein unsubstituiertes oder substituiertes Phenyl, wo die Substituenten ein oder zwei der gleichen oder verschiedenen von Chlor, Fluor, Brom, Jod, Nitro, Methyl, Ethyl, Methoxy oder Trifluormethvl sein können, oder |

ein unsubstituiertes Furyl oder Thienyl oder ein unsubstituiertes oder substituiertes Pyrrolyl, wo der Substituent ($C_1$-$C_4$)Alkyl sein kann, sind

wobei A oder B ein unsubstituierter oder wie vorstehend angegebener substituierter, 5-gliedriger Heterozyklus ist.

**5.** Verwendung nach Anspruch 1, worin die Insektizid-aktive Verbindung eine oder mehrere der Verbindungen
N'-t-Butyl-N-benzoyl-N'-(2-thiophencarbonyl)hydrazin,
N'-t-Butyl-N-(3-thiophencarbonyl)-N'-benzoylhydrazin und
N'-t-Butyl-N-(3-furoyl)-N'-benzoylhydrazin umfaßt.

**6.** Verwendung einer insektiziden Verbindung nach einem der Ansprüche 1 bis 5, gegebenenfalls in einem auch für ein landwirtschaftlich verträgliches Verdünnungsmittel oder einen Träger enthaltenden Mittel zur Insektenkontrolle, bei der die Insekten mit einer wirksamen Menge der Verbindung oder des Mittels in Kontakt gebracht werden.

**7.** Verwendung nach einem der vorhergehenden Ansprüche, worin die vorliegende Verbindung 0,001 bis etwa 90 Gew.-%, vorzugsweise 0,01 bis etwa 75 Gew.-% des Mittels beträgt.

**8.** Verwendung nach einem der vorhergehenden Ansprüche, worin das insektizide Mittel enthält (a) ein Dispersionsmittel, wobei das Mittel in Form eines benetzbaren Pulvers vorliegt, (b) einen flüssigen landwirtschaftlich verträglichen Träger und ein Dispersionsmittel, wobei das Mittel in fließbarer Form oder (c) in Form von Staub vorliegt, (d) ein Bindemittel, wobei das Mittel in Form von Granulat vorliegt, (e) einen Lockstoff, wobei das Mittel in Form eines Köders vorliegt, oder (f) ein Emulgiermittel, wobei das Mittel in Form eines emulgierbaren Konzentrats vorliegt.

**9.** Verwendung nach einem der Ansprüche 6 bis 8, worin die Verbindung oder das Mittel wachsenden Pflanzen oder dem Wachstumsmedium, in dem Pflanzen wachsen sollen, mit einer Rate von 10 g bis etwa 10 kg der wirksamen Verbindung pro Hektar, vorzugsweise 100 g bis 5 kg pro Hektar verabreicht wird.

**10.** Verfahren nach Anspruch 9, worin die Insekten der Ordnung Lepidoptera angehören.

**Revendications**
**Revendications pour les Etats contractants suivants : BE, CH, DE, FR, GB, GR, IT, LI, LU, NL, SE**

**1.** Un composé insecticidement actif qui est une diacylhydrazine substituée de formule

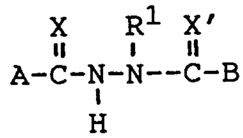

dans laquelle

X et X' sont, les mêmes ou différents, O, S ou NR;

$R^1$ est un alcoyl ramifié en $C_3$-$C_{10}$ non substitué ou un alcoyl à chaîne droite en $C_1$-$C_4$ substitué par un ou deux, les mêmes ou différents, cycloalcoyl en $C_3$-$C_6$ ; et

A et B sont phényl non substitué ou substitué dans lequel les substituants peuvent être de un à cinq, les mêmes ou différents, halo ; nitro ; cyano ; hydroxy ; alcoyl en $C_1$-$C_6$ ; haloalcoyl en $C_1$-$c_6$ ; cyanoalcoyl en $C_1$-$C_6$ ; alcoxy en $C_1$-$C_6$ ; haloalcoxy en $C_1$-$C_6$ ; alcoxyalcoyl ayant indépendamment 1 à 6 atomes de carbone dans chaque groupe alcoyl ; alcoxyalcoxy ayant indépendamment 1 à 6 atomes de carbone dans chaque groupe alcoyl ; groupe $-OCO_2R$ ; alcényl en $C_2$-$C_6$ éventuellement substitué par halo, cyano, alcoyl en $C_1$-$C_4$, alcoxy en $C_1$-$C_4$, haloalcoxy en $C_1$-$C_4$ ou alcoylthio en $C_1$-$C_4$ ; alcényl (en $C_2$-$C_6$)-carbonyl ; alcadiényl en $C_2$-$C_6$ ; alcynyl en $C_2$-$C_6$ éventuellement substitué par halo, cyano, nitro, hydroxy, alcoyl en $C_1$-$C_4$, alcoxy en $C_1$-$C_4$, haloalcoxy en $C_1$-$C_4$ ou alcoylthio en $C_1$-$C_4$ ; carboxy ; groupe $-ZCO_2R'$; -COR' ;haloalcoxyl (en $C_1$-$C_6$)-carbonyl ; cyanoalcoyl (en $C_1$-$C_6$)-carbonyl; nitroalcoyl (en $C_1$-$C_6$)-carbonyl; alcoxy-carbonyl en $C_1$-$C_6$ ; haloalcoxy (en $C_1$ à $C_6$)-carbonyl ; groupe -OCOR ; groupe -NRR' ; amino substitué par hydroxy, groupes alcoxy en $C_1$-$C_4$ ou alcylthio en $C_1$-$C_4$ ; groupe -CONRR' ; groupe -OCONRR' ; groupe -NRCOR'; groupe $-NRCO_2R'$; groupe -OCONRCOR' ; sulfhydryl ; alcoylthio en $C_1$-$C_6$ ; haloalcoylthio en $C_1$ à $C_6$ ; groupe -NRCSR' ; groupe -SCOR;

ou phényl non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxy dans lequel le noyau phénylique est non substitué ou substitué par un à trois, les mêmes ou différents, halo, cyano, nitro, hydroxy, alcoyl en $C_1$-$C_4$, alcoxy en $C_1$-

$C_4$, çarboxy, alcoxy (en $C_1$-$C_4$)-carbonyl, alcanoyloxy en $C_1$-$C_4$ ou amino ;

ou benzoyl dans le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxycarbonyl dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénylthio dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou, lorsque deux positions adjacents sur le noyau phénylique sont substituées par des groupes alcoxy, ces groupes peuvent être réunis pour former, avec les atomes de carbone auxquels ils sont attachés, un noyau hétérocyclique dioxolano ou dioxano à 5 ou 6 constituants ; ou un hétérocycle à cinq éléments, non substitué ou substitué, choisi parmi furyl, thiényl, triazolyl, pyrrolyl, isopyrrolyl, pyrazolyl, isoimidazolyl, thiazolyl, isothiazolyl, oxazolyl et isooxazolyl, dans lesquels les substituants peuvent être de un à trois, les mêmes ou différents, halo; nitro ; hydroxy ; alcoyl en $C_1$-$C_6$; alcoxy en $C_1$-$C_6$ ; carboxy ; alcoxy (en $C_1$-$C_6$)-carbonyl ; groupe -$ZCO_2R'$; groupe -CONRR' ; groupe -NRR' ; groupe -NRCOR' ; alcoylthio en $C_1$-$C_6$ ; ou phényl non substitué ou substitué par un à trois, les mêmes ou différents, halo, nitro, alcoyl en $C_1$-$C_6$, haloalcoyl en $C_1$-$C_6$, alcoxy en $C_1$-$C_6$, haloalcoxy en $C_1$-$C_6$, carboxy, alcoxy (en $C_1$-$C_4$)-carbonyl ou amino (-NRR') ;
avec la condition que l'un de A ou B soit un hétérocycle à cinq éléments, non substitué ou substitué, tel que défini ci-dessus, dans lequel R et R' sont hydrogène ou alcoxyl en $C_1$-$C_6$ ; Z est alcoyl en $C_1$-$C_6$ ; "amino" signifie NRR' ;

ou un sel agronomiquement acceptable de celle-ci.

**2.** Un composé selon la revendication 1, dans lequel

X et X' sont O ou S ;

$R^1$ est un alcoyl ramifié en $C_3$-$C_8$ ; et

A et B sont phényl non substitué ou substitué par un à trois, les mêmes ou différents, halo ; nitro ; cyano ; alcoyl en $C_1$-$C_4$ ; haloalcoyl en $C_1$-$C_4$ ; cyanoalcoyl en $C_1$-$C_4$ ; alcoxy en $C_1$-$C_4$ ; alcoxyalcoyl ayant indépendamment 1 à 4 atomes de carbone dans chaque groupe alcoyl ; -COD ; alcoxy (en $C_1$-$C_4$)-carbonyl ; alcanoyloxy en $C_1$-$C_4$ ; phényl non substitué ou substitué par un à deux, les mêmes ou différents halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou -NDD' ; ou phénoxy dans lequel le noyau phénylique est non substitué ou substitué par un ou deux, les mêmes ou différents, halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$, ou -NDD' ; ou
un hétérocycle à cinq éléments, non substitué ou substitué, choisi parmi furyl, thiényl, triazolyl, pyrrolyl et oxazolyl, dans lesquels les substituants peuvent être un ou deux, les mêmes ou différents, halo ; nitro ; alcoyl en $C_1$-$C_4$ ; alcoxy en $C_1$-$C_4$ ; -NDD' ; ou phényl non substitué ou substitué par un ou deux, les mêmes ou différents, halo, nitro, alcoyl en $C_1$-$C_4$, haloalcoyl en $C_1$-$C_4$, alcoxy en $C_1$-$C_4$, haloalcoxy en $C_1$-$C_4$, carboxy ou -NDD' ;
un de A ou B étant un hétérocycle, non substitué ou substitué, à cinq éléments comme défini ci-dessus, dans lequel D et D' sont hydrogène ou alcoyl en $C_1$-$C_4$.

**3.** Un composé selon la revendication 2, dans lequel

X et X' sont O ;

$R^1$ est un alcoyl ramifié en $C_4$-$C_7$ ; et

A et B sont phényl ou phényl substitué dans lequel les substituants peuvent être de un à trois, les mêmes ou différents, halo, nitro, alcoyl en $C_1$-$C_4$, alcoxy en $C_1$-$C_4$ ou haloalcoyl en $C_1$-$C_4$ ; ou
un hétérocycle à cinq éléments, non substitué ou substitué, choisi parmi furyl, thiényl, pyrrolyl et oxazolyl, dans lesquels les substituants peuvent être un ou deux, les mêmes ou différents, halo, nitro, alcoyl en $C_1$-$C_4$, alcoxy en $C_1$-$C_4$ ou haloalcoyl en $C_1$-$C_4$ ; et
un de A ou B étant un hétérocycle à cinq éléments, non substitué ou substitué, comme défini ci-dessus.

**4.** Un composé selon la revendication 3,dans lequel

X et X' sont O ;

$R^1$ est t-butyl, néopentyl (2,2-diméthylpropyl) ou 1,2,2-triméthylpropyl ; et

A et B sont phényl ou phényl substitué dans lequel les substituants peuvent être un ou deux, les mêmes ou différents, chloro, fluoro, bromo, iodo, nitro, méthyl, éthyl, méthoxy ou trifluorométhyl ; ou furyl ou thiényl non substitué ou un pyrrolyl non substitué ou substitué dans lequel le substituant peut être alcoyl en C1-C4, un de A ou B étant un hétérocycle à cinq éléments, non substitué ou substitué, comme défini ci-dessus.

**5.** Les composés insecticides selon la revendication 1 :

N'-t-butyl-N-benzoyl-N'-(2-thiophènecarbonyl)hydrazine,

N'-t-butyl-N-(3-thiophènecarbonyl)-N'-benzoyl hydrazine et

N'-t-butyl-N-(3-furoyl)-N'-benzoylhydrazine.

**6.** Une composition insecticide comprenant un véhicule diluant agronomiquement acceptable et un composé insecticide selon l'une quelconque des revendications 1 à 5.

**7.** Une composition selon la revendication 6, dans laquelle ledit composé est présent en une teneur de 0,001 à environ 90%, de préférence de 0,01 à environ 75%, en poids, de la composition.

**8.** Une composition insecticide selon la revendication 6 ou 7 (a) contenant un agent dispersant, ladite composition étant sous la forme d'une poudre mouillable, ou (b) contenant un véhicule liquide agronomiquement acceptable et un agent dispersant, ladite composition étant sous la forme d'un produit coulant, ou (c) sous la forme d'une poussière, ou (d) contenant un agent de liaison, ladite composition étant sous la forme d'un granulé ou (e) contenant un agent attractif, ladite composition étant sous la forme d'un appât, ou (f) contenant un agent émulsifiant, ladite composition étant sous la forme d'un concentré émulsifiable.

**9.** Une méthode pour contrôler les insectes qui comprend la mise en contact desdits insectes avec un composé insecticide selon l'une quelconque des revendications 1 à 5, éventuellement sous la forme d'une composition selon l'une quelconque des revendications 6 à 8.

**10.** Une méthode selon la revendication 9, dans laquelle ledit composé ou ladite composition est appliqué à des plantes en culture ou au milieu de culture dans lesquelles les plantes sont cultivées ou vont être cultivées, en une quantité d'environ 10 grammes à 10 kilogrammes de composé actif par hectare, de préférence de 100 grammes à 5 kilogrammes par hectare.

**11.** Une méthode selon la revendication 10, dans laquelle lesdits insectes font partie de l'ordre des Lépidoptères.

**Revendications pour l'Etat contractant suivant : AT**

**1.** Une composition insecticide comprenant un composé insecticidement actif qui est une diacylhydrazine substituée de formule

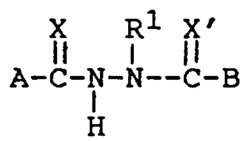

dans laquelle

X et X' sont, les mêmes ou différents, O, S ou NR;

$R^1$ est un alcoyl ramifié en $C_3$-$C_{10}$ non substitué ou un alcoyl à chaîne droite en $C_1$-$C_4$ substitué par un ou deux, les mêmes ou différents, cycloalcoyl en $C_3$-$C_6$ ; et

A et B sont phényl non substitué ou substitué dans lequel les substituants peuvent être de un à

cinq, les mêmes ou différents, halo ; nitro ; cyano ; hydroxy ; alcoyl en $C_1$-$C_6$ ; haloalcoyl en $C_1$-$C_6$ ; cyanoalcoyl en $C_1$-$C_6$ ; alcoxy en $C_1$-$C_6$ ; haloalcoxy en $C_1$-$C_6$ ; alcoxyalcoyl ayant indépendamment 1 à 6 atomes de carbone dans chaque groupe alcoyl ; alcoxyalcoxy ayant indépendamment 1 à 6 atomes de carbone dans chaque groupe alcoyl ; groupe $-OCO_2R$ ; alcényl en $C_2$-$C_6$ éventuellement substitué par halo, cyanc, alcoyl en $C_1$-$C_4$, alcoxy en $C_1$-$C_4$, haloalcoxy en $C_1$-$C_4$ ou alcoylthio en $C_1$-$C_4$ ; alcényl (en $C_2$-$C_6$)-carbonyl ; alcadiényl en $C_2$-$C_6$ ; alcynyl en $C_2$-$C_6$ éventuellement substitué par halo, cyano, nitro, hydroxy, alcoyl en $C_1$-$C_4$, alcoxy en $C_1$-$C_4$, haloalcoxy en $C_1$-$C_4$ ou alcoylthio en $C_1$-$C_4$ ; carboxy ; groupe $-ZCO_2R'$; -COR' ;haloalcoxyl (en $C_1$-$C_6$)-carbonyl ; cyanoalcoyl (en $C_1$-$C_6$)-carbonyl; nitroalcoyl (en $C_1$-$C_6$)-carbonyl; alcoxy-carbonyl en $C_1$-$C_6$ ; haloalcoxy (en $C_1$ à $C_6$)-carbonyl ; groupe -OCOR ; groupe -NRR' ; amino substitué par hydroxy, groupes alcoxy en $C_1$-$C_4$ ou alcylthio en $C_1$-$C_4$ ; groupe -CONRR' ; groupe -OCONRR' ; groupe -NRCOR'; groupe $-NRCO_2R'$; groupe -OCONRCOR' ; sulfhydryl ; alcoylthio en $C_1$-$C_6$ ; haloalcoylthio en $C_1$ à $C_6$; groupe -NRCSR' ; groupe -SCOR;

ou phényl non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxy dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou benzoyl dans le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxycarbonyl dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénylthio dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou, lorsque deux positions adjacents sur le noyau phénylique sont substituées par des groupes alcoxy, ces groupes peuvent être réunis pour former, avec les atomes de carbone auxquels ils sont attachés, un noyau hétérocyclique dioxolano ou dioxano à 5 ou 6 constituants ; ou un hétérocycle à cinq éléments, non substitué ou substitué, choisi parmi furyl, thiényl, triazolyl, pyrrolyl, isopyrrolyl, pyrazolyl, isoimidazolyl, thiazolyl, isothiazolyl, oxazolyl et isooxazolyl, dans lesquels les substituants peuvent être de un à trois, les mêmes ou différents, halo; nitro ; hydroxy ; alcoyl en $C_1$-$C_6$; alcoxy en $C_1$-$C_6$ ; carboxy ; alcoxy (en $C_1$-$C_6$)-carbonyl ; groupe $-ZCO_2R'$; groupe -CONRR' ; groupe -NRR' ; groupe -NRCOR' ; alcoylthio en $C_1$-$C_6$ ; ou phényl non substitué ou substitué par un à trois, les mêmes ou différents, halo, nitro, alcoyl en $C_1$-$C_6$, haloalcoyl en $C_1$-$C_6$, alcoxy en $C_1$-$C_6$, haloalcoxy en $C_1$-$C_6$, carboxy, alcoxy (en $C_1$-$c_4$)-carbonyl ou amino (-NRR') ;

avec la condition que l'un de A ou B soit un hétérocycle à cinq éléments, non substitué ou substitué, tel que défini ci-dessus, dans lequel R et R' sont hydrogène ou alcoxyl en $C_1$-$c_6$ ; Z est alcoyl en $C_1$-$C_6$ ; "amino" signifie NRR' ;

ou un sel agronomiquement acceptable de celle-ci.

**2.** Une composition selon la revendication 1, dans laquelle

X et X' sont O ou S ;

$R^1$ est un alcoyl ramifié en $C_3$-$C_8$ ; et

A et B sont phényl non substitué ou substitué par un à trois, les mêmes ou différents, halo ; nitro ; cyano ; alcoyl en $C_1$-$C_4$ ; haloalcoyl en $C_1$-$C_4$ ; cyanoalcoyl en $C_1$-$C_4$ ; alcoxy en $C_1$-$C_4$ ; alcoxyalcoyl ayant indépendamment 1 à 4 atomes de carbone dans chaque groupe alcoyl ; -COD ; alcoxy (en $C_1$-$C_4$)-carbonyl ; alcanoyloxy en $C_1$-$C_4$ ; phényl non substitué ou substitué par un à deux, les mêmes ou différents halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou -NDD' ; ou phénoxy dans lequel le noyau phénylique est non substitué ou substitué par un ou deux, les mêmes ou différents, halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$, ou -NDD' ; ou

un hétérocycle à cinq éléments, non substitué ou substitué, choisi parmi furyl, thiényl,

triazolyl, pyrrolyl et oxazolyl, dans lesquels les substituants peuvent être un ou deux, les mêmes ou différents, halo ; nitro ; alcoyl en $C_1$-$C_4$ ; alcoxy en $C_1$-$C_4$ ; -NDD' ; ou phényl non substitué ou substitué par un ou deux, les mêmes ou différents, halo, nitro, alcoyl en $C_1$-$C_4$, haloalcoyl en $C_1$-$C_4$, alcoxy en $C_1$-$C_4$, haloalcoxy en $C_1$-$C_4$, carboxy ou -NDD' ;
un de A ou B étant un hétérocycle, non substitué ou substitué, à cinq éléments comme défini ci-dessus, dans lequel D et D' sont hydrogène ou alcoyl en $C_1$-$C_4$.

**3.** Une composition selon la revendication 2, dans laquelle
X et X' sont O ;
$R^1$ est un alcoyl ramifié en $C_4$-$C_7$ ; et
A et B sont phényl ou phényl substitué dans lequel les substituants peuvent être de un à trois, les mêmes ou différents, halo, nitro, alcoyl en $C_1$-$C_4$, alcoxy en $C_1$-$C_4$ ou haloalcoyl en $C_1$-$C_4$ ; ou
un hétérocycle à cinq éléments,non substitué ou substitué, choisi parmi furyl, thiényl, pyrrolyl et oxazolyl, dans lesquels les substituants peuvent être un ou deux, les mêmes ou différents, halo, nitro, alcoyl en $C_1$-$C_4$, alcoxy en $C_1$-$C_4$ ou haloalcoyl en $C_1$-$C_4$ ; et
un de A ou B étant un hétérocycle à cinq éléments, non substitué ou substitué, comme défini ci-dessus.

**4.** Une composition selon la revendication 3, dans laquelle
X et X' sont O ;
$R^1$ est t-butyl, néopentyl (2,2-diméthylpropyl) ou 1,2,2-triméthylpropyl ; et
A et B sont phényl ou phényl substitué dans lequel les substituants peuvent être un ou deux, les mêmes ou différents, chloro, fluoro, bromo, iodo, nitro, méthyl, éthyl, méthoxy ou trifluorométhyl ; ou
furyl ou thiényl non substitué ou un pyrrolyl non substitué ou substitué dans lequel le substituant peut être alcoyl en C1-C4,
un de A ou B étant un hétérocycle à cinq éléments, non substitué ou substitué, comme défini ci-dessus.

**5.** Une composition selon la revendication 1, dans laquelle le composé insecticidement actif comprend un ou plusieurs de :
N'-t-butyl-N-benzoyl-N'-(2-thiophènecarbonyl)hydrazine,
N'-t-butyl-N-(3-thiophènecarbonyl)-N'-benzoyl hydrazine et
N'-t-butyl-N-(3-furoyl)-N'-benzoylhydrazine.

**6.** Une composition selon une quelconque revendication précédente, dans laquelle ledit composé est présent en une teneur de 0,001 à environ 90%, de préférence de 0,01 à environ 75%, en poids, de la composition.

**7.** Une composition insecticide selon une quelconque revendication précédente (a) contenant un agent dispersant, ladite composition étant sous la forme d'une poudre mouillable, ou (b) contenant un véhicule liquide agronomiquement acceptable et un agent dispersant, ladite composition étant sous la forme d'un produit coulant, ou (c) sous la forme d'une poussière, ou (d) contenant un agent de liaison, ladite composition étant sous la forme d'un granulé ou (e) contenant un agent attractif, ladite composition étant sous la forme d'un appât, ou (f) contenant un agent émulsifiant, ladite composition étant sous la forme d'un concentré émulsifiable.

**8.** Une méthode pour contrôler les insectes qui comprend la mise en contact desdits insectes avec un composé insecticide tel que défini dans l'une quelconque des revendications 1 à 5, éventuellement sous la forme d'une composition selon une quelconque revendication précédente.

**9.** Une méthode selon la revendication 8, dans laquelle ledit composé ou ladite composition est appliqué à des plantes en culture ou au milieu de culture dans lesquelles les plantes sont cultivées ou vont être cultivées, en une quantité d'environ 10 grammes à 10 kilogrammes de composé actif par hectare, de préférence de 100 grammes à 5 kilogrammes par hectare.

**10.** Une méthode selon la revendication 9, dans laquelle lesdits insectes font partie de l'ordre des

Lépidoptères.

**Revendications pour l'Etat contractant suivant : ES**

1. L'utilisation d'un composé insecticidement actif qui est une diacylhydrazine substituée de formule

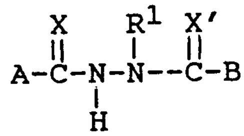

dans laquelle

X et X' sont, les mêmes ou différents, O, S ou NR;

$R^1$ est un alcoyl ramifié en $C_3$-$C_{10}$ non substitué ou un alcoyl à chaîne droite en $C_1$-$C_4$ substitué par un ou deux, les mêmes ou différents, cycloalcoyl en $C_3$-$C_6$ ; et

A et B sont phényl non substitué ou substitué dans lequel les substituants peuvent être de un à cinq, les mêmes ou différents, halo ; nitro ; cyano ; hydroxy ; alcoyl en $C_1$-$C_6$ ; haloalcoyl en $C_1$-$C_6$ ; cyanoalcoyl en $C_1$-$C_6$ ; alcoxy en $C_1$-$C_6$ ; haloalcoxy en $C_1$-$C_6$ ; alcoxyalcoyl ayant indépendamment 1 à 6 atomes de carbone dans chaque groupe alcoyl ; alcoxyalcoxy ayant indépendamment 1 à 6 atomes de carbone dans chaque groupe alcoyl ; groupe $-OCO_2R$ ; alcényl en $C_2$-$C_6$ éventuellement substitué par halo, cyano, alcoyl en $C_1$-$C_4$, alcoxy en $C_1$-$C_4$, haloalcoxy en $C_1$-$C_4$ ou alcoylthio en $C_1$-$C_4$ ; alcényl (en $C_2$-$C_6$)-carbonyl ; alcadiényl en $C_2$-$C_6$ ; alcynyl en $C_2$-$C_6$ éventuellement substitué par halo, cyano, nitro, hydroxy, alcoyl en $C_1$-$C_4$, alcoxy en $C_1$-$C_4$, haloalcoxy en $C_1$-$C_4$ ou alcoylthio en $C_1$-$C_4$ ; carboxy ; groupe $-ZCO_2R'$; -COR' ;haloalcoxyl (en $C_1$-$C_6$)-carbonyl ; cyanoalcoyl (en $C_1$-$C_6$)-carbonyl; nitroalcoyl (en $C_1$-$C_6$)-carbonyl; alcoxy-carbonyl en $C_1$-$C_6$ ; haloalcoxy (en $C_1$ à $C_6$)-carbonyl ; groupe -OCOR ; groupe -NRR' ; amino substitué par hydroxy, groupes alcoxy en $C_1$-$C_4$ ou alcylthio en $C_1$-$C_4$ ; groupe -CONRR' ; groupe -OCONRR' ; groupe -NRCOR'; groupe $-NRCO_2R'$ ; groupe -OCONRCOR' ; sulfhydryl ; alcoylthio en $C_1$-$C_6$ ; haloalcoylthio en $C_1$ à $C_6$; groupe -NRCSR' ; groupe -SCOR;

ou phényl non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxy dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou benzoyl dans le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénoxycarbonyl dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou phénylthio dans lequel le noyau phénylique est non substitué ou substitué par un à trois, les mêmes ou différents, halo, cyano, nitro, hydroxy, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou amino ;

ou, lorsque deux positions adjacents sur le noyau phénylique sont substituées par des groupes alcoxy, ces groupes peuvent être réunis pour former, avec les atomes de carbone auxquels ils sont attachés, un noyau hétérocyclique dioxolano ou dioxano à 5 ou 6 constituants ; ou un hétérocycle à cinq éléments, non substitué ou substitué, choisi parmi furyl, thiényl, triazolyl, pyrrolyl, isopyrrolyl, pyrazolyl, isoimidazolyl, thiazolyl, isothiazolyl, oxazolyl et isooxazolyl, dans lesquels les substituants peuvent être de un à trois, les mêmes ou différents, halo; nitro ; hydroxy ; alcoyl en $C_1$-$C_6$; alcoxy en $C_1$-$C_6$ ; carboxy ; alcoxy (en $C_1$-$C_6$)-carbonyl ; groupe $-ZCO_2R'$; groupe -CONRR' ; groupe -NRR' ; groupe -NRCOR' ; alcoylthio en $C_1$-$C_6$ ; ou phényl non substitué ou substitué par un à trois, les mêmes ou différents, halo, nitro, alcoyl en $C_1$-$C_6$, haloalcoyl en $C_1$-$C_6$, alcoxy en $C_1$-$C_6$, haloalcoxy en $C_1$-$C_6$, carboxy, alcoxy (en $C_1$-$C_4$)-carbonyl ou amino (-NRR') ; avec la condition que l'un de A ou B soit un hétérocycle à cinq éléments, non substitué

ou substitué, tel que défini ci-dessus, dans lequel R et R' sont hydrogène ou alcoxyl en $C_1$-$C_6$ ; Z est alcoyl en $C_1$-$C_6$ ; "amino" signifie NRR' ;
ou d'un sel agronomiquement acceptable de celle-ci.

**2.** L'utilisation d'un composé selon la revendication 1, dans lequel

X et X' sont O ou S ;
$R^1$ est un alcoyl ramifié en $C_3$-$C_8$ ; et
A et B sont phényl non substitué ou substitué par un à trois, les mêmes ou différents, halo ; nitro ; cyano ; alcoyl en $C_1$-$C_4$ ; haloalcoyl en $C_1$-$C_4$ ; cyanoalcoyl en $C_1$-$C_4$ ; alcoxy en $C_1$-$C_4$ ; alcoxyalcoyl ayant indépendamment 1 à 4 atomes de carbone dans chaque groupe alcoyl ; -COD ; alcoxy (en $C_1$-$C_4$)-carbonyl ; alcanoyloxy en $C_1$-$C_4$ ; phényl non substitué ou substitué par un à deux, les mêmes ou différents halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$ ou -NDD' ; ou phénoxy dans lequel le noyau phénylique est non substitué ou substitué par un ou deux, les mêmes ou différents, halo, nitro, alcoyl en $C_1$-$C_4$, alcoxy en $C_1$-$C_4$, carboxy, alcoxy (en $C_1$-$C_4$)-carbonyl, alcanoyloxy en $C_1$-$C_4$, ou -NDD' ; ou
un hétérocycle à cinq éléments, non substitué ou substitué, choisi parmi furyl, thiényl, triazolyl, pyrrolyl et oxazolyl, dans lesquels les substituants peuvent être un ou deux, les mêmes ou différents, halo ; nitro ; alcoyl en $C_1$-$C_4$ ; alcoxy en $C_1$-$C_4$ ; -NDD' ; ou phényl non substitué ou substitué par un ou deux, les mêmes ou différents, halo, nitro, alcoyl en $C_1$-$C_4$, haloalcoyl en $C_1$-$C_4$, alcoxy en $C_1$-$C_4$, haloalcoxy en $C_1$-$C_4$, carboxy ou -NDD' ;
un de A ou B étant un hétérocycle, non substitué ou substitué, à cinq éléments comme défini ci-dessus, dans lequel D et D' sont hydrogène ou alcoyl en $C_1$-$C_4$.

**3.** L'utilisation selon la revendication 2, dans laquelle

X et X' sont O ;
$R^1$ est un alcoyl ramifié en $C_4$-$C_7$ ; et
A et B sont phényl ou phényl substitué dans lequel les substituants peuvent être de un à trois, les mêmes ou différents, halo, nitro, alcoyl en $C_1$-$C_4$, alcoxy en $C_1$-$C_4$ ou haloalcoyl en $C_1$-$C_4$ ; ou
un hétérocycle à cinq éléments,non substitué ou substitué, choisi parmi furyl, thiényl, pyrrolyl et oxazolyl, dans lesquels les substituants peuvent être un ou deux, les mêmes ou différents, halo, nitro, alcoyl en $C_1$-$C_4$, alcoxy en $C_1$-$C_4$ ou haloalcoyl en $C_1$-$C_4$ ; et
un de A ou B étant un hétérocycle à cinq éléments, non substitué ou substitué, comme défini ci-dessus.

**4.** L'utilisation selon la revendication 3, dans laquelle

X et X' sont O ;
$R^1$ est t-butyl, néopentyl (2,2-diméthylpropyl) ou 1,2,2-triméthylpropyl ; et
A et B sont phényl ou phényl substitué dans lequel les substituants peuvent être un ou deux, les mêmes ou différents, chloro, fluoro, bromo, iodo, nitro, méthyl, éthyl, méthoxy ou trifluorométhyl ; ou
furyl ou thiényl non substitué ou un pyrrolyl non substitué ou substitué dans lequel le substituant peut être alcoyl en C1-C4,
un de A ou a étant un hétérocycle à cinq éléments, non substitué ou substitué, comme défini ci-dessus.

**5.** L'utilisation selon la revendication 1, dans laquelle le composé insecticide comprend un ou plusieurs de :

N'-t-butyl-N-benzoyl-N'-(2-thiophènecarbonyl)hydrazine,
N'-t-butyl-N-(3-thiophènecarbonyl)-N'-benzoyl hydrazine et
N'-t-butyl-N-(3-furoyl)-N'-benzoylhydrazine.

**6.** L'utilisation d'un composé insecticide tel que défini dans l'une quelconque des revendications 1 à 5, éventuellement dans une composition contenant un diluant ou véhicule agronomiquement acceptable, pour contrôler des insectes par la mise en contact desdits insectes avec une quantité efficace du composé ou de la composition.

**7.** L'utilisation selon une quelconque revendication précédente, dans laquelle ledit composé est présent en une teneur de 0,001 à environ 90%, de préférence de 0,01 à environ 75%, en poids, de la composition.

**8.** L'utilisation selon une quelconque revendication précédente, dans laquelle ladite composition insecticide (a) contient un agent dispersant, ladite composition étant sous la forme d'une poudre mouillable, ou (b) contient un véhicule liquide agronomiquement acceptable et un agent dispersant, ladite composition étant sous la forme d'un produit coulant, ou (c) est sous la forme d'une poussière, ou (d) contient un agent de liaison, ladite composition étant sous la forme d'un granulé ou (e) contient un agent attractif, ladite composition étant sous la forme d'un appat, ou (f) contient un agent émulsifiant, ladite composition étant sous la forme d'un concentré émulsifiable.

**9.** L'utilisation selon l'une quelconque des revendications 6 à 8, dans laquelle ledit composé ou ladite composition est appliqué à des plantes en culture ou au milieu de culture dans lesquelles les plantes sont cultivées ou vont être cultivées, en une quantité d'environ 10 grammes à 10 kilogrammes de composé actif par hectare, de préférence de 100 grammes à 5 kilogrammes par hectare.

**10.** L'utilisation selon la revendication 9, dans laquelle lesdits insectes font partie de l'ordre des Lépidoptères.